(19) Europäisches Patentamt
European Patent Office
Office européen des brevets

(11) EP 0 591 040 B1

(12) **FASCICULE DE BREVET EUROPEEN**

(45) Date de publication et mention de la délivrance du brevet:
**03.02.1999 Bulletin 1999/05**

(51) Int. Cl.$^6$: **C07D 453/02**, A61K 31/445

(21) Numéro de dépôt: **93402362.3**

(22) Date de dépôt: **28.09.1993**

(54) **Amides basiques quaternaires comme antagonistes des tachykinines**

Quartäre basische Amide als Antagonisten von Tachykininen

Quaternary basic amides as tachykinines antagonists

(84) Etats contractants désignés:
**AT BE CH DE DK ES FR GB GR IE IT LI LU NL PT SE**

(30) Priorité: **30.09.1992 FR 9212083**

(43) Date de publication de la demande:
**06.04.1994 Bulletin 1994/14**

(73) Titulaire: **SANOFI**
**75013 Paris (FR)**

(72) Inventeurs:
- **Emonds-Alt, Xavier**
  **F-34980 Combaillaux (FR)**
- **Gueule, Patrick**
  **F-34820 Teyran (FR)**
- **Proietto, Vincenzo**
  **F-34680 St Georges d'Orques (FR)**
- **Van Broeck, Didier**
  **F-34570 Murviel les Montpellier (FR)**

(74) Mandataire:
**Gillard, Marie-Louise et al**
**Cabinet Beau de Loménie**
**158, rue de l'Université**
**75340 Paris Cédex 07 (FR)**

(56) Documents cités:
**EP-A- 0 428 434** **EP-A- 0 474 561**

Remarques:
Le dossier contient des informations techniques présentées postérieurement au dépôt de la demande et ne figurant pas dans le présent fascicule.

EP 0 591 040 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet de nouveaux amides basiques quaternaires, un procédé pour leur préparation et les compositions pharmaceutiques les contenant en tant que principes actifs.

Plus particulièrement, la présente invention concerne une nouvelle classe d'amides basiques quaternaires à usage thérapeutique, dans les phénomènes pathologiques qui impliquent le système des tachykinines comme par exemple de manière non limitative et exclusive : la douleur (D. Regoli et al., Life Sciences, 1987, *40*, 109-117), l'allergie et l'inflammation (J.E. Morlay et al., Life Sciences, 1987, *41*, 527-544), l'insuffisance circulatoire (J. Losay et al., 1977, Substance P, Von Euler, U.S. and Pernow ed., 287-293, Raven Press, New York), les troubles gastro-intestinaux (D. Regoli et al., Trends Pharmacol. Sci., 1985, *6*, 481-484), les troubles respiratoires (J. Mizrahi et al., Pharmacology, 1982, *25*, 39-50).

Des ligands endogènes aux récepteurs des tachykinines ont été décrits, telles la substance P (SP), la neurokinine A ($NK_A$) (S.J. Bailey et al., 1983, Substance P, P. Skrabanck ed., 16-17 Boole Press, Dublin) et la neurokinine B ($NK_B$) (S.P. Watson, Life Sciences, 1983, *25*, 797-808).

Ainsi selon un de ses aspects, la présente invention concerne des amides basiques quaternaires de formule :

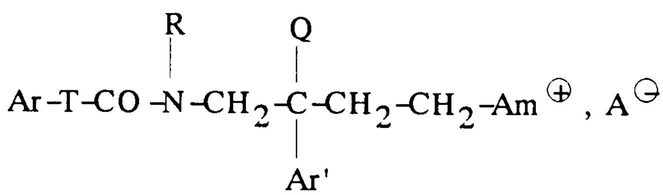

(I)

dans laquelle

- Ar représente un groupe aromatique ou hétéroaromatique mono-, di- ou tricyclique éventuellement substitué tel que défini ci-après ;
- T représente une liaison directe, un groupe hydroxyméthylène, un groupe alcoxyméthylène dont le groupe alcoxy est en $C_1$-$C_4$ ou un groupe alkylène en $C_1$-$C_5$;
- Ar' représente un phényle non substitué ou substitué une ou plusieurs fois par un des substituants choisis parmi : un atome d'halogène, de préférence un atome de chlore ou de fluor, un trifluorométhyle, un alcoxy en $C_1$-$C_4$, un alkyle en $C_1$-$C_4$, lesdits substituants étant identiques ou différents ; un thiényle ; un benzothiényle ; un naphtyle ou un indolyle ;
- R représente l'hydrogène ; un alkyle en $C_1$-$C_4$ ; un ω-($C_1$-$C_4$)alcoxy($C_2$-$C_4$)alkyle; ou un ω-($C_2$-$C_4$)alcanoyloxy($C_2$-$C_4$)alkyle ;
- Q représente l'hydrogène ;
- ou bien Q et R, ensemble, forment un groupe 1,2-éthylène, 1,3-propylène ou 1,4-butylène ;
- $Am^{\oplus}$ représente le radical

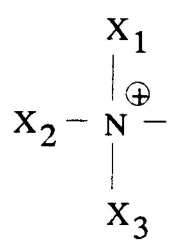

dans lequel $X_1$, $X_2$, $X_3$, ensemble et avec l'atome d'azote auquel ils sont liés, forment un système azabicyclique choisi parmi 1-azoniabicyclo[2.2.1]heptane et 1-azoniabicyclo[2.2.2]octane, éventuellement substitué par un groupe phényle ou benzyle ;
- $A^{\ominus}$ est un anion pharmaceutiquement acceptable.

Les anions pharmaceutiquement acceptables sont ceux normalement utilisés pour salifier les ions d'ammonium quaternaires de produits à utilisation pharmaceutique, de préférence les ions chlorure, bromure, iodure, hydrogénosulfate, méthanesulfonate, paratoluènesulfonate, acétate, benzènesulfonate.

Dans la formule (I), Ar représente un groupe aromatique ou hétéroaromatique mono-, di- ou tricyclique, pouvant porter un ou plusieurs substituants, dont en particulier un atome de carbone du carbocycle aromatique ou de l'hétérocycle aromatique est directement lié à T, choisi parmi un groupe phényle, qui peut être non substitué ou éventuellement contenir un ou plusieurs substituants, de préférence mono- ou disubstitué notamment en position 2,4, mais aussi par exemple en position 2,3, 4,5, 3,4 ou 3,5 ; il peut aussi être trisubstitué, notamment en position 2,4,6 mais aussi par exemple en 2,3,4, 2,3,5 ou 2,4,5 3,4,5 ; tétrasubstitué, par exemple en 2,3,4,5 ; ou pentasubstitué. Les substituants du groupe phényle peuvent être : F ; Cl ; Br ; I ; CN ; OH ; $NH_2$ ; NH-$CONH_2$ ; $NO_2$ ; $CONH_2$ ; $CF_3$ ; alkyle en $C_1$-$C_{10}$, de préférence en $C_1$-$C_4$, méthyle ou éthyle étant préférés, ainsi que par exemple n-propyle, isopropyle, n-butyle, isobutyle, sec-butyle, tert-butyle, pentyle ou n-pentyle, hexyle ou n-hexyle, octyle ou n-octyle, nonyle ou n-nonyle ainsi que décyle ou n-décyle ; alcényle contenant de 2 à 10, de préférence 2-4 atomes de carbone, par exemple vinyle, allyle, 1-propényle, isopropényle, butényle ou 1-butèn-1-, -2-, -3- ou -4-yle, 2-butèn-1-yle, 2-butèn-2-yle, pentényle, hexényle ou décényle ; alcynyle contenant de 2 à 10, de préférence 2-4 atomes de carbone, par exemple éthynyle, 1-propyn-1-yle, propargyle, butynyle ou 2-butyn-1-yle, pentynyle, décynyle ; cycloalkyle contenant de 3 à 8, de préférence 5 ou 6 atomes de carbone, cyclopentyle ou cyclohexyle étant préférés, ainsi que par exemple cyclopropyle, cyclobutyle, 1-, 2- ou 3-méthylcyclopentyle, 1-, 2-, 3- ou 4-méthylcyclohexyle, cycloheptyle ou cyclooctyle ; bicycloalkyle contenant de 4 à 11, de préférence 7 atomes de carbone, exo ou endo 2-norbornyle étant préférés, ainsi que par exemple 2-isobornyle ou 5-camphyle ; hydroxyalkyle contenant de 1 à 5, de préférence 1-2 atomes de carbone, hydroxyméthyle et 1- ou 2-hydroxyéthyle étant préférés, ainsi que par exemple 1-hydroxyprop-1-yle, 2-hydroxyprop-1-yle, 3-hydroxyprop-1-yle, 1-hydroxyprop-2-yle, 1-hydroxybut-1-yle, 1-hydroxypent-1-yle ; alcoxy contenant de 1 à 10, de préférence 1-4 atomes de carbone, isopropoxy ou éthoxy étant préférés, ainsi que par exemple n-propoxy, méthoxy, n-butoxy, isobutoxy, sec-butoxy, tert-butoxy, pentyloxy, hexyloxy, heptyloxy, octyloxy, nonyloxy ou décyloxy ; alcoxyalkyle contenant de 2 à 10, de préférence de 2 à 6 atomes de carbone, par exemple alcoxyméthyle ou alcoxyéthyle, tel que méthoxyméthyle ou 1- ou 2-méthoxyéthyle, 1- ou 2-n-butoxyéthyle, 1- ou 2-n-octyloxyéthyle ; alcoxyalcoxyalkyle contenant jusqu'à 10, de préférence de 4 à 7 atomes de carbone, par exemple alcoxyalcoxyméthyle,par exemple 2-méthoxyéthoxyméthyle, 2-éthoxyéthoxy-méthyle ou 2-isopropoxyéthoxyméthyle, alcoxyalcoxyéthyle par exemple 2-(2-méthoxyéthoxy) éthyle ou 2-(2-éthoxyéthoxy)éthyle ; alcoxyalcoxy contenant de 2 à 10, de préférence de 3 à 6 atomes de carbone, par exemple 2-méthoxy-éthoxy, 2-éthoxyéthoxy ou 2-n-butoxyéthoxy ; alcényloxy contenant de 2 à 10, de préférence 2 à 4 atomes de carbone, allyloxy étant préféré, ainsi que par exemple vinyloxy, propényloxy, isopropényloxy, buténlyloxy tel que 1-butèn-1-, -2-, -3- ou -4-yloxy, 2-butèn-1-yloxy, 2-butèn-2-yloxy, pentènyloxy, hexényloxy ou décényloxy ; alcényloxyalkyle avec jusqu'à 10, de préférence 3-6 atomes de carbone, par exemple allyloxyméthyle; alcynyloxy contenant de 2 à 10, de préférence 2 à 4 atomes de carbone, propargyloxy étant préféré, ainsi que par exemple éthynyloxy, 1-propyn-1-yloxy, butynyloxy ou 2-butyn-1-yloxy, pentynyloxy ou décynyloxy ; alcynyloxyalkyle contenant de 3 à 10 de préférence 3 à 6 atomes de carbone, par exemple éthynyloxyméthyle, propargyloxyméthyle ou 2-(2-butyn-1-yloxy)éthyle ; cycloalcoxy contenant de 3 à 8, de préférence 5 ou 6 atomes de carbone, cyclopentyloxy ou cyclohexyloxy étant préférés, ainsi que par exemple cyclopropyloxy, cyclobutyloxy, 1-, 2- ou 3-méthylcyclopentyloxy, 1-, 2-, 3- ou 4-méthylcyclohexyloxy, cycloheptyloxy ou cyclooctyloxy ; alkylthio contenant de 1 à 10, de préférence 1 à 4 atomes de carbone, méthylthio ou éthylthio étant préférés, ainsi que par exemple n-propylthio, isopropylthio, n-butylthio, isobutylthio, sec-butylthio, tert-butylthio, pentylthio, hexylthio, octylthio, nonylthio ou décylthio ; alkylthioalkyle contenant de 2 à 10, de préférence 2 à 6 atomes de carbone, par exemple méthylthiométhyle, 2-méthylthioéthyle ou 2-n-butylthioéthyle ; acylamino, à savoir alcanoylamino contenant de 1 à 7, de préférence 1 à 4 atomes de carbone, formylamino et acétylamino étant préférés, ainsi que propionylamino, butyrylamino, isobutyrylamino, valérylamino, caproylamino, heptanoylamino, ainsi que benzoylamino ; acylaminoalkyle, de préférence alcanoylaminoalkyle contenant de 2 à 8, de préférence 3 à 6 atomes de carbone, tel que formylaminoéthyle, acétylaminoéthyle, propionylaminoéthyle, n-butyrylaminoéthyle, formylaminopropyle, acétylaminopropyle, propionylamino-propyle, formylaminobutyle, acétylaminobutyle, ainsi que propionylaminobutyle, butyrylaminobutyle ; acyloxy contenant de 1 à 6, de préférence 2 à 4 atomes de carbone, acétyloxy, propionyloxy ou butyryloxy étant préférés, ainsi que par exemple formyloxy, valéryloxy, caproyloxy ; alcoxycarbonyle contenant de 2 à 5, de préférence 2 et 3 atomes de carbone, méthoxycarbonyle et éthoxycarbonyle étant préférés, ainsi que par exemple n-propoxycarbonyle, isopropoxycarbonyle, n-butoxycarbonyle, isobutoxycarbonyle, sec-butoxycarbonyle ou tert-butoxycarbonyle ; cycloalcoxycarbonyle contenant de 4 à 8, de préférence 6 ou 7 atomes de carbone, cyclopentyloxycarbonyle, cyclohexyloxycarbonyle étant préférés, ainsi que cyclopropyloxycarbonyle, cyclobutyloxycarbonyle ou cycloheptyloxycarbonyle ; alkylaminocarbonylamino contenant de 2 à 4 atomes de carbone, tel que méthylaminocarbonylamino, éthylaminocarbonylamino, propylaminocarbonylamino ; dialkylaminocarbonylamino contenant de 3 à 7, de préférence 3 à 5 atomes de carbone, de préférence diméthylaminocarbonylamino, ainsi que di-n-propylaminocarbonylamino, diisopropylaminocarbonylamino ; (1-pyrrolidino)-carbonylamino ; cycloalkylaminocarbonylamino contenant de 4 à 8, de préférence 6 ou 7 atomes de carbone, cyclopentylaminocarbonylamino, cyclohexylaminocarbonylamino étant préférés, ainsi que cyclopropylaminocarbonylamino, cyclobutylaminocarbonylamino, cycloheptyl-aminocarbonylamino; alkylaminocarbonylaminoalkyle contenant de 3 à 9, de préférence 4 à 7 atomes de

carbone, méthylaminocarbonylaminoéthyle, éthylamino-carbonylaminoéthyle, éthylaminocarbonylaminopropyle, éthylaminocarbonylaminobutyle étant préférés, ainsi que par exemple méthylaminocarbonylaminométhyle, n-propylaminocarbonylaminobutyle, n-butylaminocarbonylaminobutyle ; dialkylamino-carbonylaminoalkyle contenant de 4 à 11 atomes de carbone, par exemple diméthylaminocarbonylaminométhyle, diéthylaminocarbonylaminoéthyle, diéthylaminocarbonylaminopropyle, diéthylaminocarbonylaminobutyle, (1-pyrrolidino) carbonylaminoéthyle, (1-pipéridino)- carbonylaminoéthyle, cycloalkylaminocarbonylaminoalkyle contenant de 5 à 12, de préférence 8 à 11 atomes de carbone, cyclopentylaminocarbonyl-aminoéthyle, cyclopentylaminocarbonylaminopropyle, cyclopentylaminocarbonyl-aminobutyle, cyclohexylaminocarbonylaminoéthyle, cyclohexylaminocarbonylaminopropyle et cyclohexylaminocarbonylaminobutyle étant préférés, ainsi que par exemple cyclopropylaminocarbonylaminoéthyle, cycloheptylaminocarbonylaminoéthyle ; alcoxycarbonylaminoalkyle contenant de 3 à 12, de préférence 4 à 9 atomes de carbone, méthoxycarbonylaminoéthyle, éthoxycarbonylaminoéthyle, n-propoxycarbonyleaminoéthyle, isopropoxycarbonylaminoéthyle, n-butoxycarbonyl-aminoéthyle, isobutoxy-carbonylaminoéthyle, sec-butoxycarbonylaminoéthyle, tert-butoxycarbonylamino-éthyle, éthoxycarbonylaminopropyle, n-butoxycarbonylaminopropyle, éthoxycarbonylaminobutyle, n-butoxycarbonylaminobutyle étant préférés, ainsi que par exemple n-propoxycarbonylaminopropyle, n-propoxy-carbonylaminobutyle, isopropoxycarbonylaminobutyle ; cycloalcoxycarbonylaminoalkyle contenant de 5 à 12, de préférence 8 à 11 atomes de carbone, cyclopentyloxycarbonylaminoéthyle, cyclopentyloxycarbonylaminopropyle, cyclopentyoxycarbonylaminobutyle, cyclohexyloxycarbonylaminoéthyle, cyclohexyloxycarbonylaminopropyle, cyclohexyloxycarbonylaminobutyle étant préférés, ainsi que par exemple cyclopropyloxycarbonylaminométhyle, cycloheptyloxycarbonylaminoéthyle ; carbamoylalkyle contenant de 2 à 5, de préférence 2 atomes de carbone, de préférence carbamoylméthyle, ainsi que carbamoyléthyle, carbamoylpropyle, carbamoylbutyle ; alkylaminocarbonylalkyle contenant de 3 à 9, de préférence 3 à 6 atomes de carbone, méthylaminocarbonyléthyle, éthylaminocarbonylméthyle, n-propylaminocarbonylméthyle, isopropylaminocarbonylméthyle, n-butylaminocarbonylméthyle, isobutylaminocarbonylméthyle, sec-butylaminocarbonylméthyle, tert-butylaminocarbonylméthyle étant préférés, ainsi que par exemple éthylaminocarbonyléthyle, éthylaminocarbonylpropyle, éthylaminocarbonylbutyle, propylaminocarbonylbutyle, n-butylaminocarbonylbutyle ; dialkylaminocarbonylalkyle contenant de 4 à 11, de préférence 4 à 8 atomes de carbone, diméthylaminocarbonylméthyle, diéthylaminocarbonylméthyle, di-n-propylaminocarbonylméthyle ; ainsi que par exemple diéthylaminocarbonyléthyle, diéthylaminocarbonylpropyle, diéthylaminocarbonybutyle; (1-pyrrolidine)-carbonylméthyle, (1-pipéridino)carbonylméthyle ; (1-pipéridino)-carbonyléthyle ; cycloalkyl-aminocarbonylalkyle contenant de 5 à 12, de préférence 7 ou 8 atomes de carbone, cyclopentylaminocarbonylméthyle et cyclohexylaminocarbonylméthyle étant préférés, ainsi que par exemple cyclopropylaminocarbonylméthyle, cyclobutyl-aminocarbonylméthyle, cycloheptylaminocarbonylméthyle, cyclohexylamino-carbonyléthyle, cyclohexylaminocarbonylpropyle, cyclohexylaminocarbonylbutyle ; alkylaminocarbonylalcoxy contenant de 3 à 10, de préférence 3 à 5 atomes de carbone, méthylaminocarbonylméthoxy étant préféré, ainsi que par exemple méthylaminocarbonyléthoxy, méthylaminocarbonylpropoxy ; dialkylaminocarbonylalcoxy contenant de 4 à 10, de préférence 4 à 7 atomes de carbone, tel que diméthylaminocarbonylméthoxy, diéthylaminocarbonyléthoxy, (pipéridinyl-1) carbonylméthoxy ; cycloalkylaminocarbonylalcoxy contenant de 5 à 11, de préférence 7 ou 8 atomes de carbone, tel que cyclopentylaminocarbonylméthoxy, cyclohexylaminocarbonylméthoxy.

Le radical Ar peut également représenter un groupe aromatique bicyclique 1- ou 2-naphtyle ; 1-, 2-, 3-, 4-, 5-, 6-, 7-indényle ; dont une ou plusieurs liaisons peuvent être hydrogénées, lesdits groupes pouvant être non substitués ou contenir éventuellement un ou plusieurs substituants choisis parmi : un halogène et plus particulièrement un atome de fluor, les groupes alkyle, phényle, cyano, hydroxyalkyle, hydroxy, alcoxy, oxo, alkylcarbonylamino, alcoxycarbonyle et thioalkyle dans lesquels les alkyles sont en $C_1$-$C_4$.

Le radical Ar peut être aussi un groupe pyridyle, thiadiazolyle, indolyle, indazolyle, imidazolyle, benzimidazolyle, quinoléyle, benzotriazolyle, benzofuranyle, benzothiényle, benzothiazolyle, benzisothiazolyle, isoquinolyle, benzoxazolyle, benzoxazinyle, benzodioxinyle, isoxazolyle, benzopyranyle, thiazolyle, thiényle, furyle, pyranyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromanyle, chromanyle, dont une ou plusieurs doubles liaisons peuvent être hydrogénées, lesdits groupes pouvant être non substitués ou contenir éventuellement un ou plusieurs substituants choisis parmi : les groupes alkyle, phényle, cyano, hydroxyalkyle, hydroxy, alkylcarbonylamino, alcoxycarbonyle et thioalkyle dans lesquels les alkyles sont en $C_1$-$C_4$.

De manière avantageuse, le radical Ar représente un phényle non substitué ou substitué une ou plusieurs fois par un atome d'halogène, plus particulièrement un atome de chlore ou de fluor, un trifluorométhyle, un alkyle en $C_1$-$C_4$, un hydroxyle, un alcoxy en $C_1$-$C_4$; un naphtyle non substitué ou substitué une ou plusieurs fois par un halogène, un trifluorométhyle, un alkyle en $C_1$-$C_4$, un hydroxyle, un alcoxy en $C_1$-$C_4$ ; un pyridyle ; un thiényle ; un indolyle un quinoléyle ; un benzothiényle ; un imidazolyle.

Les composés particulièrement préférés sont ceux de formule (I) dans laquelle Ar est un groupe phényle substitué par un groupe isopropoxy, avantageusement en position 3.

Dans la formule (I), T représente de préférence un groupe méthylène.

Les substituants R et Q sont, de préférence, respectivement un groupe méthyle et l'hydrogène ; un groupe 2-méthoxyéthyle et l'hydrogène ; un groupe 2-acétoxyéthyle et l'hydrogène ou R et Q, ensemble, forment un groupe 1,3-propylène.

Le substituant Ar' est de préférence un groupe phényle, avantageusement substitué par deux atomes de chlore, plus particulièrement dans les positions 3 et 4.

Le radical

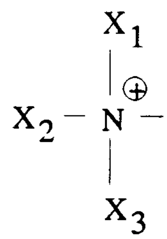

représenté par le substituant $Am^{\oplus}$ dans la formule (I) est de préférence le résidu d'un système azabicyclique choisi parmi : 1-azoniabicyclo[2.2.2]octane (d) et 4-phényl-1-azoniabicyclo[2.2.2]octane (l).

Des amides basiques quaternaires particulièrement préférés selon la présente invention sont ceux de formule (I) dans laquelle à la fois :

- Ar est un groupe 3-isopropoxyphényle ;
- T est un groupe méthylène ;
- R et Q sont, respectivement,un groupe méthyle et l'hydrogène; un groupe 2-acétoxyéthyle et l'hydrogène ; ou bien, ensemble, forment un groupe 1,3-propylène ;
- Ar' est 3,4-dichlorophényle ;
- $Am^{\oplus}$ est un radical (d) ou (l) tels que définis ci-dessus ;
- $A^{\ominus}$ est un anion pharmaceutiquement acceptable, le chlorure, le méthanesulfonate ou le benzènesulfonate de façon préférentielle.

Ces produits, représentés par la formule :

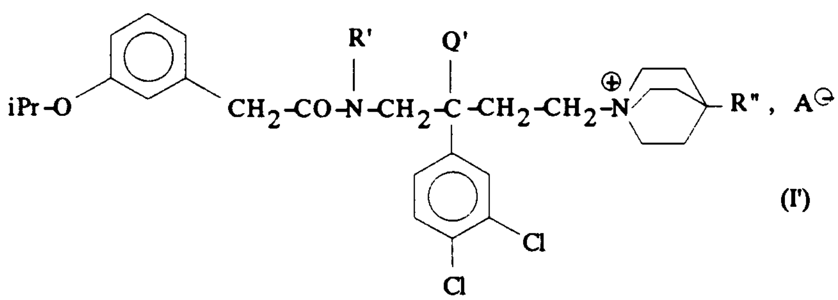

(I')

dans laquelle iPr représente isopropyle, R' et Q' représentent, respectivement, un groupe méthyle et l'hydrogène; un groupe 2-acétoxyéthyle et l'hydrogène ou, ensemble, forment un groupe 1,3-propylène, R" est l'hydrogène ou un groupe phényle et $A^{\ominus}$ est tel que défini ci-dessus, notamment l'ion méthanesulfonate ou chlorure, sont des puissants antagonistes de la substance P.

Les composés de formule (I') dans laquelle R' et Q', ensemble, forment un groupe 1,3-propylène sont extrêmement puissants et montrent une affinité pour le récepteur de la neurokinine 1 supérieure à celle de la Substance P elle-même. Ils constituent donc l'aspect préférentiel de la présente invention.

Parmi ces composés, ceux de formule (I") :

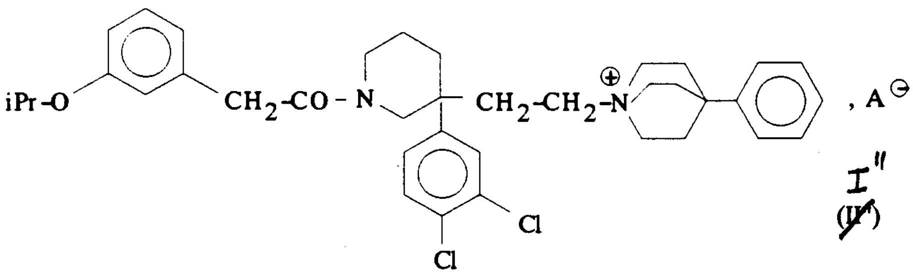

I''

dans laquelle A$^{\ominus}$ est un anion pharmaceutiquement acceptable, notamment le méthanesulfonate, le chlorure et le benzènesulfonate sont les plus intéressants.

Selon un autre de ses aspects, la présente invention concerne un procédé pour la préparation des composés de formule (I) ci-dessus, caractérisé en ce qu'on traite un dérivé de formule :

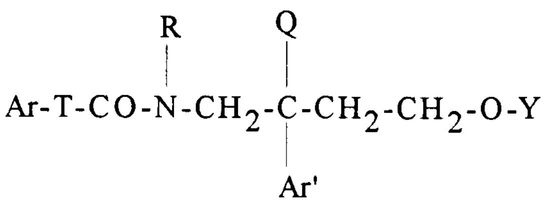

(II)

dans laquelle OY représente un groupe partant quelconque, de préférence un groupe méthanesulfonate ou benzènesulfonate avec une amine tertiaire cyclique de formule :

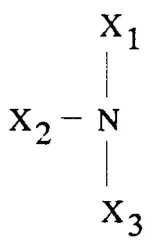

(III)

dans un solvant organique à une température comprise entre la température ambiante et 120°C et on isole le produit ainsi obtenu ou bien, éventuellement on échange l'anion méthanesulfonate du sel quaternaire ainsi obtenu avec un autre anion pharmaceutiquement acceptable.

Comme solvant organique on utilise de préférence un solvant aprotique polaire, par exemple l'acétonitrile, le N,N-diméthylformamide, le N,N-diméthylphénylacétamide, mais on peut aussi utiliser un éther, par exemple le tétrahydrofurane, le dioxane, le méthyl-t-butyléther, ou une cétone, par exemple la méthyléthylcétone, l'acétonitrile étant particulièrement préféré.

Dans la gamme de température indiquée ci-dessus, la température préférentielle est de 70-90°C. Lorsqu'on utilise l'acétonitrile comme solvant, il est avantageux d'opérer au reflux du mélange réactionnel.

Le produit ainsi obtenu est isolé selon les techniques habituelles, par exemple, par concentration des solvants puis lavage du résidu à l'eau suivi d'une purification selon les techniques conventionnelles, par exemple par chromatographie ou recristallisation.

L'anion méthanesulfonate issu de la réaction entre l'amine tertiaire de formule (III) et le dérivé méthanesulfonyloxy de formule (II) peut être échangé, in situ ou après isolement du composé (I) dans lequel A$^{\ominus}$ est l'ion méthanesulfonate, par un autre anion A$^{\ominus}$, selon les méthodes conventionnelles, par exemple par échange en solution comme une solution d'acide chlorhydrique lorsque A$^{\ominus}$ représente un anion chlorure ou par échange de l'anion par un autre anion par élution du composé (I) sur une résine échangeuse d'ion, par exemple l'Amberlite IRA68® ou Duolite A375®.

Les dérivés de formule (II) utilisés comme composé de départ pour le procédé de la présente invention peuvent être préparés selon le SCHEMA 1 ci-après selon lequel, dans les formules indiquées :

pour la **Voie A** : Q = H ; R = H , alkyle $C_1-C_4$

pour la **Voie B** : $R + Q = -(CH_2)_n-$ avec n = 2,3,4

Dans le SCHEMA 1, les réactions dans les différentes étapes sont indiquées de façon représentative afin d'indiquer le type desdites réactions sans donner les moyens mis en oeuvre, qui sont connus.

Ainsi, par exemple, dans la Voie A, étape 2, et dans la Voie B, étape 6, "$H_2$" signifie que le nitrile de départ est soumis à une réduction, par exemple à une hydrogénation catalytique (Ni-Raney dans l'éthanol en présence d'ammoniac pour obtenir l'amine primaire IV).

Dans la même étape 2 de la Voie A, le terme "alkylation" signifie qu'après la réduction l'amine primaire est soumise à une réaction d'alkylation soit directe, par un halogénure ou sulfate d'alkyle, soit indirecte, par acylation et réduction du groupe carbonyle. Ainsi, par exemple par réaction de l'amine primaire (IV) avec du chloroformiate d'éthyle et réduction du groupe éthoxycarbonyle on obtient le produit de formule (IV) où R est méthyle comme décrit dans EP-0428434 et EP-0474561. En remplaçant le chloroformiate d'éthyle par le chlorure (ou un autre dérivé fonctionnel) d'un acide alcanoique en $C_2$-$C_4$ et par réduction du groupe carbonyle du dérivé N-acylé ainsi obtenu, on prépare le composé de formule (IV) où R est alkyle en $C_2$-$C_4$.

En remplaçant le chloroformiate d'éthyle par le chlorure d'éthyloxalyle, par l'hémimalonate d'éthyle ou l'hémisuccinate d'éthyle, par exemple, on obtient les dérivés N-acylés correspondants. Les groupes carbonyles sont alors réduits selon les méthodes habituelles pour obtenir les dérivés ω-hydroxyalkyle en $C_2$-$C_4$ correspondants qui sont 0-acylés ou 0-alkylés de façon à obtenir les dérivés ω-alcanoyloxyalkylés ou ω-alkoxyalkylés de formule IV dans laquelle R est ω-($C_2$-$C_4$)-alcanoyloxy-($C_2$-$C_4$)-alkyle ou ω-($C_1$-$C_4$)-alkoxy-($C_2$-$C_4$)-alkyle.

De même, en remplaçant les chloroformiates d'alkyle par le chlorure d'un acide ω-($C_1$-$C_4$)alcoxy ($C_2$-$C_4$) alcanoïque et par réduction comme indiqué ci-dessus, on obtient directement les dérivés ω-alcoxyalkylés de formule IV où R est ω-($C_1$-$C_4$)-alkoxy-($C_2$-$C_4$)-alkyle.

Le chloroformiate d'éthyle peut être remplacé par le di-t-butylcarbonate (Boc-O-Boc) pour préparer le produit de formule IV (R = méthyle).

Egalement, par exemple, à l'étape 4 de la Voie A, "$H^+$" signifie que le groupe tétrahydropyranyloxy est soumis à une hydrolyse acide dans les conditions bien connues en littérature.

Voie A | SCHEMA 1 | Voie B

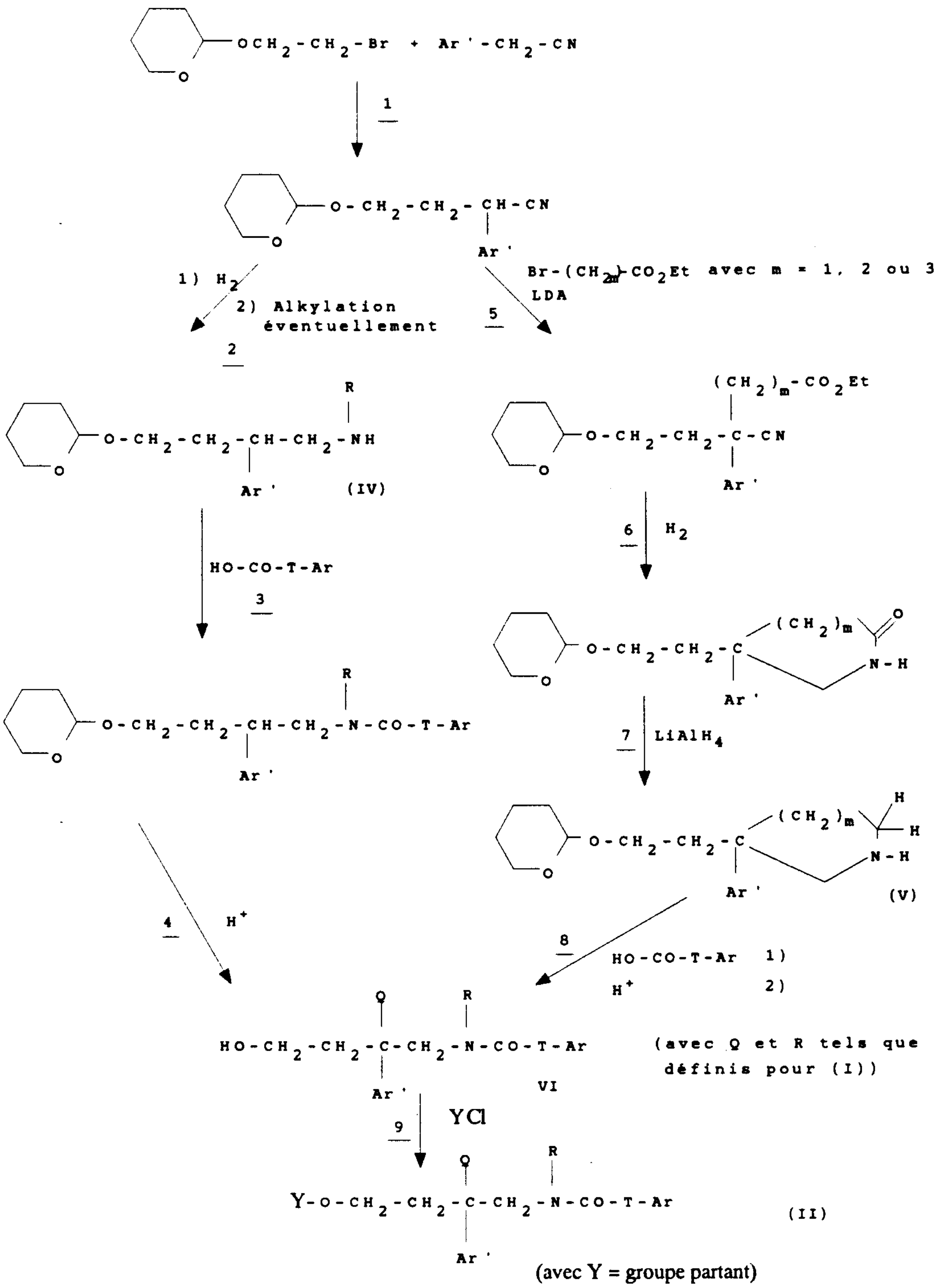


La signification du substituant Ar dépend du choix de l'acide HO-CO-T-Ar utilisé dans les étapes 3 et 8, sous forme d'un de ses dérivés fonctionnels. Tous ces acides sont bien connus dans la littérature ou facilement préparables selon la littérature ou disponibles commercialement.

La signification du substituant Ar' dépend du choix du nitrile Ar'-$CH_2$-CN qui, par réaction avec le 2-tétrahydropyranyloxy-1-bromoéthane, hydrogénation du produit ainsi obtenu et N-alkylation éventuelle (étape 2), conduit à l'amine.

La Voie A du SCHEMA 1 pour laquelle R = H, alkyle et Q = H est décrite dans la littérature, dans les demandes de brevet EP-A-0428434 et EP-A-0474561.

La Voie B du SCHEMA 1 met en oeuvre une succession de réactions bien connues de l'homme de l'art comme l'alkylation d'un nitrile par un dérivé bromé en présence de lithium diisopropylamide (LDA) (étape 5), suivie de la réduction du nitrile en présence d'un catalyseur pour obtenir l'amine correspondante après réduction de l'intermédiaire amide (étape 7) obtenu au cours de la cyclisation, (étape 6) selon par exemple, A.V. El'tsov, et al., Biol. Soedin., Akad. Nauk SSSR 1965, 109-12 (CA 1965, *63*, 16299).

Dans les deux Voies du SCHEMA 1, les conditions des réactions de certaines étapes sont les mêmes. Ainsi, la réduction des étapes 2 (Voie A) et 6 (Voie B) est faite dans les mêmes conditions. De même, l'étape 7 (Voie B) et la réduction du dérivé N-acylé ou l'éthoxycarbonyle dans la réaction d'alkylation indirecte de l'étape 2 (Voie B) se déroulent dans les mêmes conditions. Enfin, l'acylation dans les étapes 3 (Voie A) et 8 (Voie B) est effectuée dans les mêmes conditions.

Le procédé de préparation des composés (I) selon l'invention consiste à faire réagir le dérivé (II), préparé par réaction de l'alcool (IV) avec un dérivé YCl, par exemple le chlorure de mésyle ou le chlorure de benzènesulfonyle (étape 9), avec une amine tertiaire de formule (III) selon le SCHEMA 2 ci-dessous.

**SCHEMA 2**

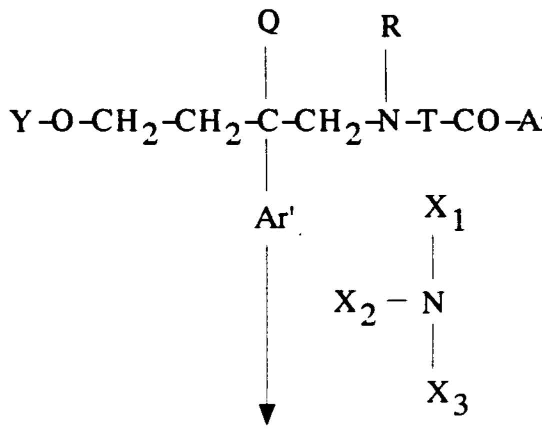

(II)

(III)

(I)

La résolution des mélanges racémiques (I) permet d'isoler les énantiomères (I*) qui font également partie de l'invention.

Il est cependant préférable d'effectuer le dédoublement des racémiques sur les aminoalcools intermédiaires susceptibles de donner des sels avec des acides optiquement actifs. Les aminoalcools correspondent aux composés (IV) et (V) obtenus selon l'étape 2 (Voie A) et selon l'étape 7 (Voie B) du SCHEMA 1 après déprotection par hydrolyse en milieu acide des composés:

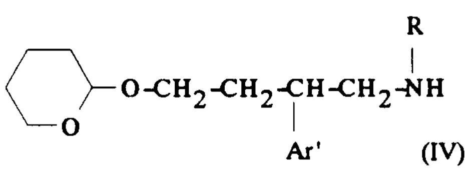

(IV) et

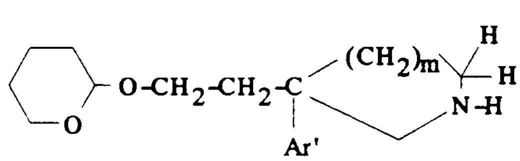

(V)

pour lesquels Ar' et R sont tels que définis pour (I) et m représente 1, 2 ou 3.

Les énantiomères sont alors séparés par des méthodes classiques telles que la cristallisation ou la chromatographie haute pression préparative chirale.

La préparation des composés optiquement purs est illustrée dans le SCHEMA 3 ci-après où "*" signifie que l'atome de carbone ainsi marqué est de configuration (+) ou (-) déterminée.

Dans le SCHEMA 3, la dernière étape est indiquée comme effectuée avec l'acide libre. Elle peut être cependant conduite avec un dérivé fonctionnel de celui-ci, qui pourrait comporter une double attaque de la molécule sur le groupe hydroxyle et sur le groupe amino. Dans ce cas il est souhaitable de procéder à une nouvelle protection du groupe hydroxyle, par exemple avec le dihydropyrane pour former le tétrahydropyranyléther.

La préparation des composés (VI*) selon le SCHEMA 3 où R est l'hydrogène ou un groupe alkyle en $C_1$-$C_4$ et où Q est l'hydrogène est décrite dans EP-A-0428434 et EP-A-0474561.

## SCHEMA 3

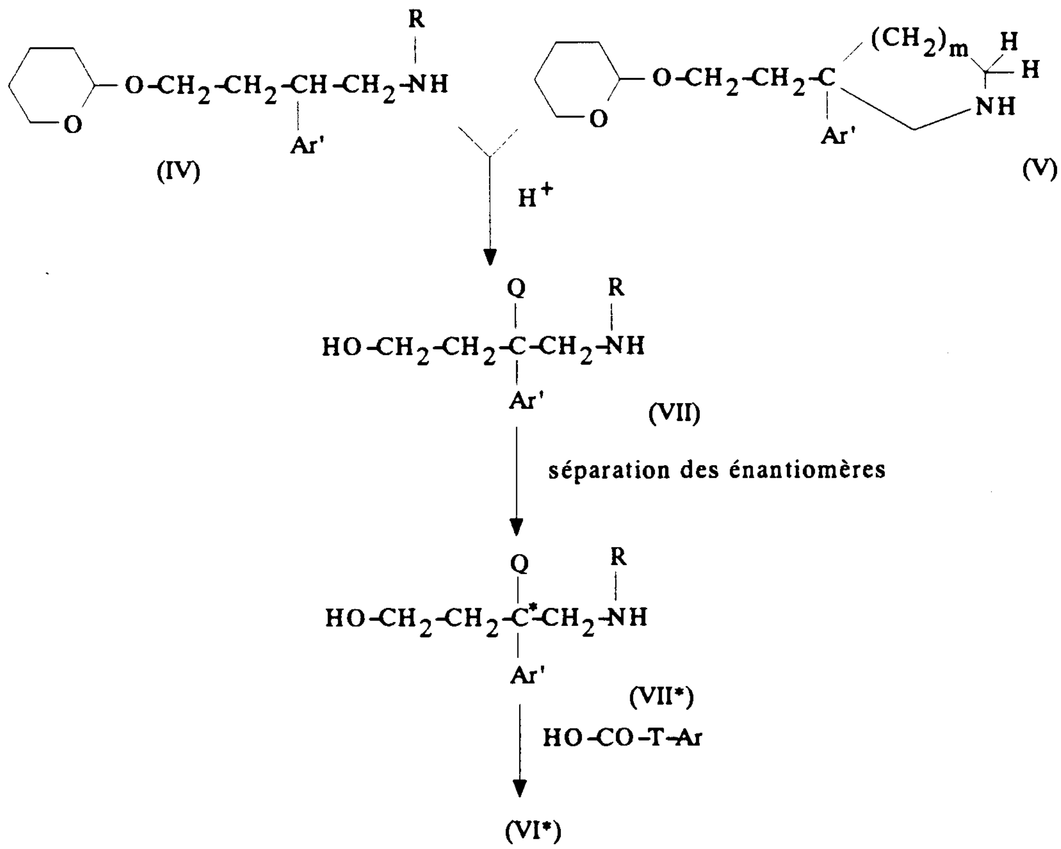

(IV) (V) (VII) (VII*) (VI*)

La préparation des composés optiquement purs de formule (VI*) dans laquelle Q et R sont liés et représentent 1,2-éthylène, 1,3-propylène ou 1,4-butylène est effectuée de la même façon.

Notamment, le composé (VII*) obtenu après séparation des énantiomères de (VII) est couplé avec un acide de formule Ar-T-COOH en présence d'un agent de couplage selon les méthodes habituelles. Comme indiqué ci-dessus, on peut également utiliser un dérivé fonctionnel de cet acide tel que l'acide lui-même, convenablement activé par exemple par le cyclohexylcarbodiimide ou par l'hexafluorophosphate de benzotriazolyl N-oxytrisdiméthylaminophosphonium (BOP), ou bien un des dérivés fonctionnels qui réagissent avec les amines, par exemple un anhydride, un anhydride mixte, le chlorure d'acide ou un ester activé, comme l'ester de paranitrophényle.

Le composé ainsi obtenu, de formule :

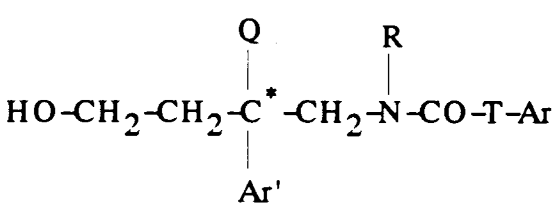
(VI*)

est alors soumis à l'action d'un dérivé YCl selon l'étape 9 du SCHEMA 1 pour conduire au dérivé (II) optiquement pur.

Les produits de formule (I) dans lesquels T représente un groupe hydroxyméthylène, alcoxyméthylène en $C_1$-$C_4$ ou alkylidène en $C_2$-$C_5$, possèdent deux centres d'asymétrie. Dans ce cas, les diastéréoisomères et les isomères purs peuvent être préparés par réaction de l'aminoalcool optiquement pur et de l'acide HO-CO-T-Ar soit optiquement pur soit racémique et, dans ce dernier cas les diastéréoisomères peuvent être séparées par exemple par chromatographie.

La réaction avec l'amine tertiaire (III) permet la préparation du produit (I) selon l'invention sous forme optiquement pure.

Les amines de formule (III) sont celles décrites dans la littérature.

Parmi ces amines, les préférées sont celles citées ci-après :

(c') 1-azabicyclo[2.2.1]heptane préparé selon Gassman et al., J. Am. Chem. Soc., 1968, (90), *5,* 1355-6.

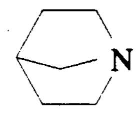

(d') 1-azabicyclo[2.2.2]octane ou quinuclidine.

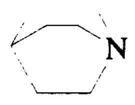

(l') 4-phényl-1-azabicyclo[2.2.2]octane, ou phényl-4 quinuclidine, préparé selon T. Perrine, J. Org. Chem., 1957, *22*, 1484-1489.

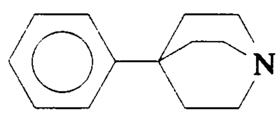

Les composés de formule (I) ci-dessus comprennent également ceux dans lesquels un ou plusieurs atomes d'hydrogène ou de carbone ont été remplacés par leur isotope radioactif par exemple le tritium ou le carbone-14. De tels composés marqués sont utiles dans des travaux de recherche, de métabolisme ou de pharmacocinétique, dans des essais biochimiques en tant que ligand de récepteurs.

Les composés selon l'invention ont fait l'objet d'essais biochimiques.

Les composés (I) ont montré des propriétés antagonistes de la liaison de la Substance P dans des essais réalisés sur des membranes de cortex de rat et de cellules lymphoblastiques IM9, selon M.A. Cascieri et al., J. Biol. Chem., 1983, *258,* 5158-5164 et D.D. Paya et al., J. Immunol., 1984, *133,* 3260-3265.

Parmi les composés testés, le chlorure de (+) 1-[2-[3-(3,4-dichlorophényl)-1-[(3-isopropoxyphényl)acétyl]-3-pipéridinyl]éthyl]-4-phényl-1azoniabicyclo[2.2.2]octane (Composé 4) s'est révélé être un puissant antagoniste du récepteur NK1 de la Substance P : il inhibe la fixation de la Substance P à son récepteur avec une constante d'inhibition (Ki) comprise entre 10-20 pM dans les différents essais biochimiques réalisés.

Les composés de la présente invention sont notamment des principes actifs de compositions pharmaceutiques, dont la toxicité est compatible avec leur utilisation en tant que médicaments.

Les composés de formule (I) ci-dessus peuvent être utilisés à des doses journalières de 0,01 à 100 mg par kilo de poids corporel du mammifère à traiter, de préférence à des doses journalières de 0,1 à 50 mg/kg. Chez l'être

humain, la dose peut varier de préférence de 0,5 à 4000 mg par jour, plus particulièrement de 2,5 à 1000 mg par jour selon l'âge du sujet à traiter ou le type de traitement : prophylactique ou curatif.

Pour leur utilisation comme médicaments, les composés de formule (I) sont généralement administrés en unité de dosage. Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I).

Dans les compositions pharmaceutiques de la présente invention pour l'administration orale, sublinguale, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange le principe actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec de liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible.

Le principe actif peut être formulé également sous forme de microcapsules, éventuellement avec un ou plusieurs supports ou additifs.

Dans chaque unité de dosage le principe actif de formule (I) est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,5 à 1000 mg de principe actif, de préférence de 2,5 à 250 mg devant être administrés une à quatre fois par jour.

Selon une autre de ses aspects, la présente invention concerne l'utilisation des produits de formule (I) pour la préparation de médicaments destinés à traiter des troubles physiologiques associés à un excès de tachykinines, notamment de Substance P et toutes les pathologies tachykinine-dépendantes du système respiratoire, gastro-intestinal, urinaire, immunitaire, cardiovasculaire et du système nerveux central ainsi que la douleur et la migraine.

Par exemple et de manière non limitative:

- douleurs aigües et chroniques liées par exemple à la migraine, aux douleurs du cancéreux et de l'angineux, aux processus inflammatoires chroniques tels que l'ostéoarthrite et l'arthrite rhumatoïde,
- les inflammations telles que les maladies respiratoires chroniques obstructives, l'asthme, les allergies, les rhinites, l'hypersensibilité par exemple pollens et acariens, arthrites rhumatoïdes, ostéoarthrites, psoriasis, colites ulcératives, maladie de Crohn, inflammation des intestins (colon irritable), prostatite, vessie neurologique, cystite, urétrite, néphrite,
- les maladies du système immunitaire liées à la suppression ou à la stimulation des fonctions des cellules immunes par exemple l'arthrite rhumatoïde, le psoriasis, la maladie de Crohn, le diabète, le lupus,
- les maladies du système nerveux central telles que l'anxiété, la dépression, la psychose, la schizophrénie, la manie, la démence, l'épilepsie, la maladie de Parkinson, la maladie d'Alzheimer, la drogue-dépendance, le syndrôme de Down et la chorée d'Huntington ainsi que les maladies neurodégénératives,

- les maladies du système gastro-intestinal telles que nausées, colon irritable, ulcères gastriques et duodénaux, diarrhées, hypersécrétions,
- les maladies du système cardiovasculaire telles que les aspects vasculaires de la migraine, oedèmes, thrombose, l'angine et les spasmes vasculaires.

La présente invention inclut aussi une méthode pour traiter lesdites affections aux doses indiquées ci-dessus.
Les exemples suivants illustrent l'invention sans toutefois la limiter.
Les points de fusion des produits, F, ont été mesurés au banc chauffant Koffler.

PREPARATIONS

A. AMINOALCOOLS (VII) et (VII*).

PREPARATION I : SCHEMA 1 - Voie A.

(a) α-(2-Tétrahydropyranyloxyéthyl)-3,4-dichlorobenzèneacétonitrile.

16,5 g d'hydrure de sodium à 80 % dans l'huile sont mis en suspension dans 200 ml de tétrahydrofurane sec. On ajoute goutte à goutte à 20°C, en 30 minutes, une solution de 100 g de 3,4-dichlorophénylacétonitrile dans 500 ml de tétrahydrofurane puis on agite le mélange réactionnel à température ambiante pendant 2 heures. Le mélange est refroidi à -20°C et on ajoute une solution de 118 g de 1-bromo-2-tétrahydropyranyl-oxyéthane dans 100 ml de tétrahydrofurane, on laisse revenir le mélange à température ambiante et après 2 heures on ajoute une solution de 50 g de chlorure d'ammonium dans 3 litres d'eau. On extrait avec 1,5 litres d'éther, lave avec une solution saturée de NaCl, décante, sèche sur $MgSO_4$, et concentre sous vide. Le résidu est chromatographié sur gel de silice, éluant : $CH_2Cl_2$ puis acétate d'éthyle 95-5 (v/v). Les fractions de produit pur sont concentrées sous vide pour fournir 118 g d'une huile.

(b) 2-(2-Tétrahydropyranyloxyéthyl)-3,4-dichlorobenzèneéthanamine.

118 g du nitrile obtenu précédemment sont mis en solution dans 700 ml d'éthanol absolu. On ajoute 300 ml d'ammoniaque concentrée puis, sous balayage d'azote, on ajoute du nickel de Raney (10 % de la quantité de nitrile de départ). On hydrogène ensuite sous atmosphère d'hydrogène à température ambiante et pression ordinaire. 16 litres sont absorbés en 4 heures. Le catalyseur est séparé par filtration sur célite, le filtrat est concentré sous vide, le résidu est repris dans une solution saturée de NaCl. Après extraction à l'éther et séchage sur $MgSO_4$, on obtient 112 g d'une huile.

(c) 2-(2-Hydroxyéthyl)-3,4-dichlorobenzèneéthanamine.

81 g du produit obtenu précédemment selon (b) sont dissous dans 38 ml de méthanol. On ajoute 80 ml d'une solution saturée d' éther chlorhydrique en maintenant la température entre 20 et 25°C. On agite pendant 30 minutes à température ambiante, puis concentre à sec. On dissous le résidu dans 250 ml d'eau, lave deux fois à l'éther, alcalinise avec une solution de NaOH, extrait au $CH_2Cl_2$. Après séchage sur $MgSO_4$, on concentre à sec, reprend dans 800 ml d' éther isopropylique, sépare un insoluble par filtration sur Célite, concentre sous vide à environ 300 ml, amorce avec des cristaux d'aminoalcool, agite pendant une nuit. On filtre, rince à l'éther isopropylique puis au n-pentane. On obtient 30,2 g du produit attendu. F = 90-91°C.

(d) 2-(2-Hydroxyéthyl)-3,4-dichlorobenzèneéthanamine (+).

A une solution bouillante de 29 g d'acide D (-) tartrique dans 800 ml de méthanol, on ajoute une solution de 44,7 g de produit obtenu selon l'étape (c) précédente dans 300 ml de méthanol. On laisse revenir à température ambiante et agite pendant 4 heures. On filtre, rince à l'éthanol puis à l'éther. On obtient 34,1 g de tartrate. On recristallise dans 1,75 l de méthanol pour obtenir 26,6 g de tartrate.

$[\alpha]_D^{25}$ = -4,2° (c = 1, dans $H_2O$)

Le tartrate est repris dans 120 ml d'eau. On alcalinise avec une solution de NaOH, extrait deux fois au $CH_2Cl_2$, sèche sur $MgSO_4$, concentre à sec. On reprend dans un peu d' éther isopropylique, ajoute du n-pentane, filtre pour obtenir 15,4 g de produit. F = 79-80°C.

$[\alpha]_D^{25}$ = + 9,4° (c = 1, dans le $CH_3OH$)

(e)Chlorhydrate de N-méthyl-2-(2-hydroxyéthyl)-3,4-dichlorobenzèneéthanamine (+)

(e1) N-[4-(2-Hydroxyéthyl)-2-(3,4-dichlorophényl)butyl]carbamate d'éthyle.

15 g de produit obtenu selon l'étape (d) précédente sont dissous dans 200 ml de $CH_2Cl_2$. On ajoute 9,9 ml de triéthylamine. On refroidit à 0°C et ajoute goutte à goutte à cette température une solution de 6,3 ml de chloroformiate d'éthyle dans 30 ml de $CH_2Cl_2$. Après 15 minutes, on lave à l'eau puis avec une solution d'HCl dilué, puis avec une solution aqueuse saturée de $NaHCO_3$. Après séchage sur $MgSO_4$, on concentre à sec pour obtenir 20 g de produit sous forme d'huile.

(e2) Réduction du groupe éthoxycarbonyle en groupe méthyle.

A 5,1 g d'hydrure de lithium/aluminium en suspension dans 60 ml de THF anhydre, on ajoute une solution de 20 g de produit obtenu selon l'étape (e1) précédente dans 150 ml de THF anhydre. On chauffe à reflux pendant 1 heure. On hydrolyse avec 20 ml d'eau, filtre le minéral, concentre à sec. L'huile obtenue est dissoute dans 100 ml d'acétone. On ajoute de l'éther saturé d'acide chlorhydrique jusqu'à pH = 1, puis de l'éther jusqu'au trouble. On agite pendant 1 heure, on filtre les cristaux, rince avec un peu d'acétone, puis d'éther pour obtenir 11 g de chlorhydrate de N-méthyl-2-(2-hydroxyéthyl)-3,4-dichlorobenzèneéthanamine. F = 129°C.

$[\alpha]_D^{25}$ = + 8,4° (c = 1, dans le $CH_3OH$)

(f)Chlorhydrate de N-méthyl-2-(2-hydroxyéthyl)-3,4-dichlorobenzèneéthanamine (-).

En procédant comme précédemment à partir de l'acide L (+) tartrique on obtient, l'énantiomère (-). F = 129°C

$[\alpha]_D^{20}$ = - 8,4° (c = 1, dans le $CH_3OH$)

PREPARATION II : SCHEMA 1 - Voie, B. m = 1.

(a) 3,4-Dichloro-α-(2-tétrahydropyranyloxyéthyl)benzèneacétonitrile.

20 g d'hydrure de sodium à 55-60 % dans l'huile sont mis en suspension dans 200 ml de tétrahydrofurane sec. On ajoute goutte à goutte à 20°C, en 30 minutes, une solution de 85 g de 3,4-dichlorophénylacétonitrile dans 500 ml de tétrahydrofurane puis on agite le mélange réactionnel à température ambiante pendant 2 heures. Au mélange ainsi obtenu, refroidi à -20°C, on ajoute une solution de 98 g de 2-bromo-éthoxytétrahydropyrane dans 100 ml de tétrahydrofurane, on laisse revenir le mélange à température ambiante et après 2 heures on ajoute une solution de 50 g de chlorure d'ammonium dans 3 litres d'eau. On extrait avec 1,5 litres d'éther éthylique, lave avec une solution saturée de chlorure de sodium, décante, sèche sur $MgSO_4$ et concentre sous vide. Le résidu est chromatographié sur gel de silice, éluant : dichlorométhane. Les fractions de produit pur sont concentrées sous vide pour fournir 83,6 g d'une huile.

(b) β-Tétrahydropyranyloxyéthyl-β-cyano-β-(3,4-dichlorophényl)propionate d'éthyle.

21 g du nitrile préparé précédemment selon (a) sont mis en solution dans 100 ml de tétrahydrofurane, puis on ajoute goutte à goutte et à température ambiante une solution de 0,067 mole de diisopropylamidure de lithium dans 100 ml de tétrahydrofurane et agite le mélange réactionnel pendant une heure à température ambiante. On ajoute alors 12 g de bromoacétate d'éthyle et chauffe à 50°C pendant deux heures. Le mélange est refroidi et on le verse dans une solution saturée de chlorure d'ammonium et extrait à l'éther éthylique, lave à l'eau, sépare la phase éthérée par décantation, la sèche sur $Na_2SO_4$ et concentre sous vide. Le résidu est purifié par chromatographie sur gel de silice, éluant : dichlorométhane/acétate d'éthyle 100/1 (v/v). La concentration des fractions pures fournit 13 g du composé attendu.

(c) 4-(2-Tétrahydropyranyloxyéthyl)-4-(3,4-dichlorophényl)-2-pyrrolidone.

13 g du composé préparé précédemment sont mis en solution dans 250 ml d'éthanol et 40 ml d'ammoniaque et sont hydrogénés à température ambiante et pression atmosphérique en présence de nickel de Raney. Lorsque le volume théorique d'hydrogène est absorbé, on filtre le mélange sur célite et on concentre le filtrat sous vide. Le résidu est repris dans l'eau, extrait à l'éther éthylique, puis on lave la phase éthérée à l'eau, la sèche sur $MgSO_4$ et concentre sous vide. On obtient ainsi 8,6 g du produit attendu.

(d) 3-(2-Tétrahydropyranyloxyéthyl)-3-(3,4-dichlorophényl)pyrrolidine.

3,9 g de 4-(2-tétrahydropyranyloxyéthyl)-4-(3,4-dichlorophényl)-2-pyrrolidone préparés précédemment sont mis en solution dans 50 ml de tétrahydrofurane et on ajoute la solution à une suspension de 0,9 g d'hydrure de lithium aluminium dans 5ml de tétrahydrofurane chauffée à 60°C . Le mélange réactionnel est chauffé pendant une heure à 60°C puis refroidi. On ajoute 1 ml d'eau, 1 ml d'hydroxyde de sodium 4N et 3 ml d'eau. On sépare le minéral par filtration et concentre le filtrat sous vide. Le résidu est repris dans l'éther éthylique, séché sur $MgSO_4$ et concentré sous vide pour fournir 3,4 g du produit attendu.

(e) 3-(2-Hydroxyéthyl)-3-(3,4-dichlorophényl)pyrrolidine.

A 3,4 g de 3-tétrahydropyranyloxyéthyl-3-(3,4-dichlorophényl)pyrrolidine en solution dans 20 ml de méthanol on ajoute de l'éther chlorhydrique jusqu'à pH = 1. On agite une demi-heure à température ambiante, concentre à sec, reprend le résidu à l'eau, basifie avec une solution d'hydroxyde de sodium, extrait au dichlorométhane, lave avec une solution saturée de NaCl, sèche sur $Na_2SO_4$ et évapore à sec. On obtient une huile. On reprend par 20 ml d'un mélange éther isopropylique/éther 50/50 (v/v). On agite, filtre, lave à l'éther éthylique et sèche sous vide sur $P_2O_5$. On isole 2,6 g du produit attendu.

PREPARATION III : SCHEMA 1 - Voie B, m = 2.

(a) $\gamma$-(2-Tétrahydropyranyloxyéthyl)-$\gamma$-cyano-$\gamma$-(3,4-dichlorophényl)butanoate d'éthyle.

21 g du nitrile préparé selon l'étape (a) précédente sont mis en solution dans 100 ml de tétrahydrofurane, puis on ajoute goutte à goutte et à température ambiante une solution de 0,067 mole de diisopropylamidure de lithium dans 100 ml de tétrahydrofurane et agite le mélange réactionnel pendant une heure à température ambiante. On ajoute alors 12 g de bromopropionate d'éthyle et chauffe à 50°C pendant deux heures. Le mélange est refroidi et on le verse dans une solution saturée de chlorure d'ammonium et extrait à l'éther, lave à l'eau, sépare la phase éthérée par décantation, la sèche sur $Na_2SO_4$ et concentre sous vide. Le résidu est purifié par chromatographie sur gel de silice, éluant : dichlorométhane/acétate d'éthyle 100/1 (v/v). La concentration des fractions pures fournit 13 g du composé attendu.

(b) 5-(2-Tétrahydropyranyloxyéthyl)-5-(3,4-dichlorophényl)-2-pipéridinone.

13 g du composé préparé précédemment sont mis en solution dans 250 ml d'éthanol et 40 ml d'ammoniaque et sont hydrogénés à température ambiante et pression atmosphérique en présence de nickel de Raney. Lorsque le volume théorique d'hydrogène est absorbé, on filtre le mélange sur célite et on concentre le filtrat sous vide. Le résidu est repris dans l'eau, extrait à l'éther, puis on lave la phase éthérée à l'eau, la sèche sur $MgSO_4$ et concentre sous vide. On obtient ainsi 9 g du produit attendu.

(c) 3-(2-Tétrahydropyranyloxyéthyl)-3-(3,4-dichlorophényl)pipéridine.

3,9 g de 5-(2-tétrahydropyranyloxyéthyl)-5-(3,4-dichlorophényl)pipéridinone préparés précédemment sont mis en solution dans 50 ml de tétrahydrofurane et on ajoute la solution à une suspension de 0,9 g d'hydrure de lithium aluminium dans 5ml de tétrahydrofurane chauffée à 60°C . Le mélange réactionnel est chauffé pendant une heure à 60°C puis refroidi. On ajoute 1 ml d'eau, 1 ml d'hydroxyde de sodium 4N et 3 ml d'eau. On élimine la partie insoluble par filtration et concentre le filtrat sous vide. Le résidu est repris dans l'éther éthylique, séché sur $MgSO_4$ et concentré sous vide pour fournir 3,4 g du produit attendu.

(d) 3-(2-Hydroxyéthyl)-3-(3,4-dichlorophényl)pipéridine.

A 55 g de 3-(2-tétrahydropyranyloxyéthyl)-3-(3,4-dichlorophényl)pipéridine en solution dans 200 ml de méthanol on ajoute de l'éther saturée d'acide chlorhydrique jusqu'à pH = 1. On agite le mélange pendant 30 minutes à température ambiante, concentre à sec, reprend le résidu à l'eau, alcalinise avec une solution de NaOH, extrait au $CH_2Cl_2$, lave avec une solution saturée de NaCl, sèche sur $Na_2SO_4$ et évapore à sec. On obtient une huile. On reprend par 200 ml d'un mélange éther isopropylique/éther 50/50 (v/v) On agite, filtre, lave à l'éther éthylique et sèche sous vide sur $P_2O_5$ et obtient 45 g du produit attendu. F = 122°C.

(e) 3-(2-Hydroxyéthyl)-3-(3,4-dichlorophényl)pipéridine (+).

A 43 g de produit obtenu précédemment en solution au reflux dans 250 ml d'éthanol 100°, on ajoute 23,54 g d'acide L (+) tartrique en solution dans 750 ml d'éthanol 100°. On chauffe le mélange réactionnel à reflux pendant une demi-heure, laisse revenir à température ambiante, on filtre les cristaux obtenus, lave à l'éthanol 100° et sèche sous vide à 50°C sur $P_2O_5$ et obtient 31 g de tartrate qui est recristallisé par mise en solution dans 540 ml d'éthanol 100°, filtration, lavage à l'éther éthylique et séchage sous vide sur $P_2O_5$ et obtient ainsi 25 g de tartrate.

$[\alpha]_D^{20}$ = + 8,5° (c = 1, dans $H_2O$)

Ce tartrate est ensuite repris dans l'eau, on neutralise avec une solution de NaOH, extrait au $CH_2Cl_2$, lave à l'eau, sèche sur $Na_2SO_4$ et évapore à sec. On reprend l'huile dans un mélange éther éthylique/éther isopropylique, filtre les cristaux, lave à l'éther éthylique et sèche sous vide à 50°C et obtient 13,5 g de base. F = 138°C

$[\alpha]_D^{20}$ = + 8,2° (c = 1, dans $CH_3OH$)

(f) 3-(2-Hydroxyéthyl)-3-(3,4-dichlorophényl)pipéridine (-).

En procédant comme précédemment à partir de l'acide D (-) tartrique on obtient, l'énantiomère (-). F = 139°C.

$[\alpha]_D^{20}$ = - 8,4° (c = 1, $CH_3OH$)

PREPARATION IV : SCHEMA 1 - Voie B, m = 3.

(a) δ-(2-Tétrahydropyranyloxyéthyl)-δ-cyano-δ-(3,4-dichlorophényl)pentanoate d'éthyle.

A 36 g de 3,4-dichloro-α-[(2-tétrahydropyranyloxy)éthyl]benzèneacétonitrile préparé selon la PREPARATION II (a) précédente, en solution dans 100 ml de diméthylformamide, on ajoute par petites portions 4,6 g de NaH à 60 %. On agite le mélange réactionnel pendant 3 heures à température ambiante, refroidit à 0°C puis on ajoute 22,4 g de 4-bromobutyrate d'éthyle dans 40 ml de diméthylformamide. On agite le mélange réactionnel pendant 3 heures à température ambiante, on verse sur l'eau, extrait à l'éther, lave avec une solution saturée NaCl, sèche sur $Na_2SO_4$ et concentre sous vide On purifie le résidu obtenu par chromatographie sur gel de silice, éluant : toluène. On obtient ainsi 24 g du produit attendu.

(b) 6-(2-Tétrahydropyranyloxyéthyl)-6-(3,4-dichlorophényl)périhydro-2-azépinone.

8 g de produit précédemment obtenu sont hydrogénés à pression atmosphérique et température ambiante en présence de nickel de Raney en solution, dans 120 ml d'éthanol. Quand le volume théorique d'hydrogène est consommé on filtre le catalyseur et concentre sous vide. L'huile obtenue est ensuite reprise dans 20 ml de xylène et le mélange réactionnel est chauffé à reflux pendant 48 heures. On évapore et purifie le résidu obtenu par chromatographie sur gel de silice, éluant : $CH_2Cl_2/CH_3OH$ 100/1 (v/v). On obtient ainsi 4 g du produit attendu sous forme d'une huile.

(c) 3-(2-Tétrahydropyranyloxyéthyl)-3-(3,4-dichlorophényl)périhydroazépine.

A partir de 2 g de produit précédemment obtenu, de 0,49 g d'hydrure de lithium aluminium et en procédant selon la préparation précédente, étape (d), on obtient 1,7 g de produit attendu sous forme d'huile.

(d) 3-(2-Hydroxyéthyl)-3-(3,4-dichlorophényl)périhydroazépine.

A partir de 1,7 g du produit obtenu précédemment et en procédant selon la préparation précédente, étape (e) on obtient 1,3 g du produit attendu.

B. ACIDES PHENYLACETIQUES SUBSTITUES.

B1. ACIDE 3-ISOPROPOXYPHENYLACETIQUE.

PREPARATION V. 1.

L'acide 3-isopropoxyphénylacétique n'est pas connu dans la littérature mais peut être préparé selon des méthodes bien connues pour la préparation d'acides alcoxyphénylacétiques.

(a) 3-Hydroxyphénylacétate d'éthyle.

55 g d'acide 3-hydroxyphénylacétique en solution dans 400 ml d'éthanol 100 sont chauffés à reflux pendant une nuit en présence de quelques gouttes d'$H_2SO_4$ concentré. On évapore à sec, reprend à l'éther éthylique, lave à l'eau puis avec une solution aqueuse saturée de $NaHCO_3$. Après séchage sur $MgSO_4$, puis évaporation on obtient 58 g d'une huile.

(b) 3-Isopropoxyphénylacétate d'éthyle.

58 g du produit obtenu précédemment, 88 g de $K_2CO_3$ et 108 g de 2-iodopropane, en solution dans 300 ml de DMF, sont chauffés à 80-100°C pendant 8 heures. On évapore le DMF sous vide, reprend à l'acétate d'éthyle et lave avec une solution aqueuse à 10 % de $K_2CO_3$. Après séchage sur $MgSO_4$ puis évaporation, on purifie le résidu par chromatographie sur gel de silice, éluant : $CH_2Cl_2$. On obtient ainsi 61 g d'une huile.

(c) Acide 3-isopropoxyphénylacétique.

31 g de produit obtenu précédemment et 20 g de NaOH en solution dans 400 ml d'éthanol, sont chauffés à reflux pendant 2 heures. On évapore à sec, reprend à l'eau et acidifie avec de l'HCl concentré. On extrait à l'éther éthylique, lave à l'eau, sèche sur $MgSO_4$ et concentre à sec pour obtenir 27 g de l'acide attendu. F = 33-35°C.

B2. ACIDE 2-IODO-5-ISOPROPOXYPHENYLACETIQUE.

PREPARATION V. 2.

L'acide 2-iodo-5-isopropoxyphénylacétique n'est pas connu dans la littérature mais peut être préparé par des méthodes connues par exemple selon R.E. Counsel et al., J. Med. Chem., 1973, *16*, *6*, 684-687 en remplaçant le chlorure de benzyle par le 2-iodopropane.
15 g de 2-iodo-5-isopropoxyphénylacétonitrile ainsi préparés sont mis en solution dans 160 ml d'éthanol en présence de 18 g de KOH puis on chauffe le mélange à reflux pendant deux heures. On concentre sous vide, reprend le résidu dans l'eau, puis successivement lave à l'éther éthylique, acidifie la phase aqueuse par addition d'HCl jusqu'à pH = 1, extrait à l'éther éthylique, lave à l'eau, sèche sur $Na_2SO_4$ et filtre. On concentre sous vide et le résidu est purifié par chromatographie sur gel de silice ; éluant : $CH_2Cl_2$-$CH_3OH$ 100-2 (v/v). On obtient après concentration des fractions pures, 8 g de l'acide attendu sous forme d'huile. Spectre de RMN (200 MHz) : 1,2 ppm - 2$CH_3$ ; 3,5 ppm - 1$CH_2$ ; 4,6 ppm - 1CH ; 6,6 ppm - 1H aromatique ; 6,9 ppm - 1H aromatique ; 7,6 ppm - 1H aromatique.

C. DERIVES ACYLES (VI) ET DERIVES SULFONYLOXY (II).

PREPARATION VI : SCHEMA 1 - Voie B, m = 1.

(a) 3-(2-Hydroxyéthyl)-3-(3,4-dichlorophényl)-1-(3-isopropoxyphényl)acétylpyrrolidine

A 1,9 g d'acide 3-isopropoxyphénylacétique en solution dans 50 ml de $CH_2Cl_2$ on ajoute 2,25 ml de triéthylamine puis 2,6 g de 3-(2-hydroxyéthyl)-3-(3,4-dichlorophényl)-pyrrolidine préparée précédemment. Le mélange est refroidi à 0°C puis on ajoute 4,42 g de BOP et laisse revenir le mélange réactionnel à température ambiante. Après 30 minutes, le mélange est concentré sous vide, le résidu est repris dans l'éther éthylique et on lave successivement à l'eau, avec une solution de NaOH diluée, avec une solution saturée de NaCl, avec une solution de HCl diluée, avec une solution saturée de NaCl et avec une solution de $NaHCO_3$. La phase éthérée est séchée sur $MgSO_4$, filtrée et concentrée sous vide pour fournir 3,6 g du produit attendu.

(b)3-(2-Méthanesulfonyloxyéthyl)-3-(3,4-dichlorophényl)-1-(3-isopropoxyphényl)-acétylpyrrolidine.

2,2 g du produit préparé précédemment sont mis en solution dans 50 ml de $CH_2Cl_2$ et on refroidit à 0°C. On ajoute 1,5 g de triéthylamine puis, goutte à goutte, 0,57 g de chlorure de méthanesulfonyle. Le mélange réactionnel est laissé 15 minutes à 0°C puis on concentre sous vide, reprend le résidu dans l'éther, lave à l'eau, sèche la phase éthérée sur $MgSO_4$, filtre et concentre sous vide. Le résidu est chromatographié sur gel de silice, éluant, heptane/acétate d'éthyle 50/50 (v/v) jusqu'à acétate d'éthyle pur. Les fractions de produit pur sont concentrées sous vide puis le résidu concrétisé dans un mélange éther éthylique/éther isopropylique pour fournir 2,5 g du produit attendu.

PREPARATION VII : SCHEMA 1 - Voie B, m = 2.

(a) 3-(2-Hydroxyéthyl)-3-(3,4-dichlorophényl)-1-[(3-isopropoxyphényl)acétyl]-pipéridine optiquement pur.

A 16 g d'acide 3-isopropoxyphénylacétique en solution dans 500 ml de $CH_2Cl_2$ on ajoute 22,5 ml de triéthylamine puis 22 g de 3-(2-hydroxyéthyl)-3-(3,4-dichlorophényl) pipéridine (-) préparé précédemment selon la PREPARATION III (f). Le mélange est refroidi à 0°C puis on ajoute 42,6 g de BOP et laisse revenir le mélange réactionnel à température ambiante. Après 30 minutes, le mélange est concentré sous vide, le résidu est repris dans l'éther et on lave successivement à l'eau, avec une solution de NaOH diluée, avec une solution saturée de NaCl, avec une solution de HCl diluée, avec une solution saturée de NaCl et avec une solution de $NaHCO_3$. La phase éthérée est séchée sur $MgSO_4$, filtrée et concentrée sous vide pour fournir 36 g de produit optiquement pur.

(b)3-(2-Méthanesulfonyloxyéthyl)-3-(3,4-dichlorophényl)-1-[(3-isopropoxyphényl)-acétyl]pipéridine (+).

36 g du produit préparé précédemment sont mis en solution dans 500 ml de $CH_2Cl_2$ et on refroidit à 0°C. On ajoute 11,5 ml de triéthylamine puis, goutte à goutte, 6,3 ml de chlorure de méthanesulfonyle. Le mélange réactionnel est laissé 15 minutes à 0°C puis on concentre sous vide, reprend le résidu dans l'éther, lave à l'eau, sèche la phase éthérée sur $MgSO_4$, filtre et concentre sous vide. Le résidu est chromatographié sur gel de silice, éluant, heptane/acétate d'éthyle 50/50 (v/v) jusqu'à acétate d'éthyle pur. Les fractions de produit pur sont concentrées sous vide puis le résidu est concrétisé dans un mélange éther éthylique/éther isopropylique pour fournir 37,5 g du produit attendu. F = 72°C

$[\alpha]_D^{20}$ = + 25,7° (c = 1, dans $CHCl_3$)

(c)3-(2-Benzènesulfonyloxyéthyl)-3-(3,4-dichlorophényl)-1-[(3-isopropoxyphényl)-acétyl]pipéridine.

A 11,3 g du produit préparé précédemment selon la PREPARATION VII (a) en solution dans 160 ml de $CH_2Cl_2$ et refroidis à 0°C on ajoute 4,6 ml de triéthylamine puis goutte à goutte 4,3 ml de chlorure de benzènesulfonyle. Le mélange réactionnel est maintenu pendant 18 heures à température ambiante puis successivement traité par 100 ml HCl, 100 ml $Na_2CO_3$ 10 %, 100 ml d'eau. La phase organique est décantée, séchée sur $Na_2SO_4$ et concentrée sous vide. Le résidu est chromatographié sur gel de silice ; éluant : cyclohexane/AcOEt 80-20 (v/v). Les fractions de produit pur sont concentrées pour fournir 8,4 g du produit attendu.

PREPARATION VIII : SCHEMA 1 - Voie B, m = 3.

(a)3-(2-Hydroxyéthyl)-3-(3,4-dichlorophényl)-1-[(3-isopropoxyphényl)acétyl]péri-hydroazépine.

A 0,76 g d'acide 3-isopropoxyphénylacétique en solution dans 50 ml de $CH_2Cl_2$ on ajoute 1,2 g de triéthylamine puis 1,15 g de 3-(2-hydroxyéthyl)-3-(3,4-dichlorophényl) azépine préparée précédemment. Le mélange est refroidi à 0°C puis on ajoute 1,77 g de BOP et laisse revenir le mélange réactionnel à température ambiante. Après 30 minutes, le mélange est concentré sous vide, le résidu est repris dans l'éther éthylique et on lave successivement à l'eau, avec une solution de NaOH diluée, avec une solution saturée de NaCl, avec une solution de HCl diluée, avec une solution saturée de NaCl et avec une solution de $NaHCO_3$. La phase éthérée est séchée sur $MgSO_4$, filtrée et concentrée sous vide pour fournir 1,8 g.

(b)3-(2-Méthanesulfonyloxyéthyl)-3-(3,4-dichlorophényl)-1-[(3-isopropoxyphényl)-acétyl]périhydroazépine.

1,8 g du produit préparé précédemment sont mis en solution dans 50 ml de $CH_2Cl_2$ et on refroidit à 0°C. On ajoute 0,38 g de triéthylamine puis, goutte à goutte, 0,44 g de chlorure de méthanesulfonyle. Le mélange réactionnel est laissé 15 minutes à 0°C puis on concentre sous vide, reprend le résidu dans l'éther éthylique, lave à l'eau, sèche la phase éthérée sur $MgSO_4$, filtre et concentre sous vide. Le résidu est chromatographié sur gel de silice, éluant, heptane/acétate d'éthyle 50/50 (v/v) jusqu'à acétate d'éthyle pur.

Les fractions de produit pur sont concentrées sous vide puis le résidu concrétisé dans un mélange éther éthylique/éther isopropylique pour fournir 2 g du produit attendu.

EXEMPLE 1

(I) : Ar = 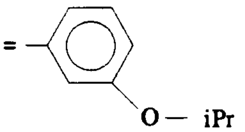 ; T = $-CH_2-$ ; R = $-CH_3$ ; Q = H ;

Ar' = 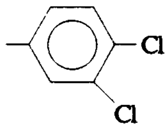 ; Am $^{\oplus}$ = 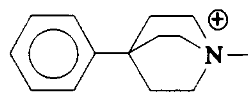 ; A $^{\ominus}$ = Cl $^{\ominus}$

0,75 g de 4-phénylquinuclidine, synthétisé selon T. Perrine, J. Org. Chem., 1957, *22,* 1484-1489, et 1 g de N-[2-(3,4-dichlorophényl)-4-méthanesulfonyloxybutyl]-N-méthyl-(3-isopropoxyphényl)carboxamide préparé selon EP-A-0428434 sont mis en solution dans 5 ml d'acétonitrile. Le mélange réactionnel est chauffé à reflux pendant 4 heures puis concentré sous vide. Le résidu est repris dans le $CH_2Cl_2$, puis successivement, on lave avec une solution HCl 2N, lave avec une solution saturée NaCl, sèche la phase organique sur $MgSO_4$, filtre et concentre sous vide. Le résidu est concrétisé dans l'éther éthylique. On obtient ainsi 0,39 g de chlorure de 1-[3-(3,4-dichlorophényl)-4-(N-méthyl-3-isopropoxyphénylacétylamino)butyl]-4-phényl-1-azoniabicyclo[2.2.2]octane (Composé 1). F = 98-100°C.

EXEMPLE 2

(I) : Ar = 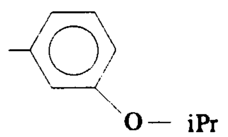 ; T = $-CH_2-$ ; R = $-CH_3$ ; Q = H ;

Ar' = 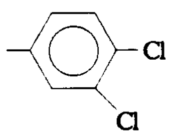 ; Am $^{\oplus}$ = 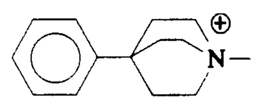 ; A $^{\ominus}$ = Cl $^{\ominus}$

En procédant selon l'Exemple 1 et en utilisant comme produit de départ le dérivé optiquement pur, (-)-N-[2-(3,4-dichlorophényl)-4-méthanesulfonyloxybutyl]-N-méthyl-(3-isopropoxyphényl)carboxamide préparé selon EP-A-428434 on obtient le dérivé du chlorure de (-) 1-[3-(3,4-dichlorophényl)-4-(N-méthyl-3-isopropoxyphénylacétylamino)butyl]-4-phényl-1-azoniabicyclo[2.2.2]octane (Composé 2), optiquement pur. F = 97-99°C.

$[\alpha]_D^{20}$ = - 47,2° (c = 1, dans $CH_3OH$)

EXEMPLE 3

(I) : Ar = 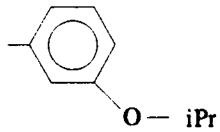 ; T = $-CH_2-$ ; R = $-CH_3$ ; Q = H ;

Ar' = 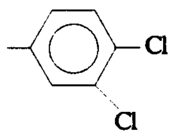 ; Am$^{\oplus}$ = 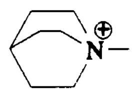 ; A$^{\ominus}$ = Cl$^{\ominus}$

En procédant selon l'Exemple 1 et en utilisant la quinuclidine comme amine tertiaire, on obtient le chlorure de 1-[3-(3,4-dichlorophényl)-4-(N-méthyl-3-isopropoxyphényl-acétylamino)butyl]-1-azoniabicyclo[2.2.2]octane (Composé 3). F = 68-70°C.

EXEMPLE 4

(I) : Ar = 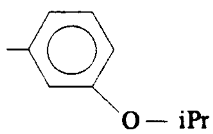 ; T = $-CH_2-$ ; R + Q = $-(CH_2)_3-$

Ar' = 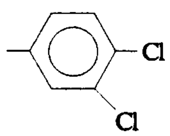 ; Am$^{\oplus}$ = 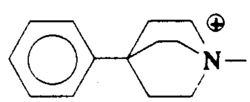 ; A$^{\ominus}$ = Cl$^{\ominus}$

2,25 g de 4-phénylquinuclidine et 3,17 g du mésylate préparé selon la PREPARATION VII (b) sont mis en solution dans 30 ml d'acétonitrile et le mélange réactionnel est chauffé à reflux pendant 10 heures. On concentre le mélange sous vide, reprend le résidu dans le $CH_2Cl_2$ et lave successivement avec une solution HCl 3N, puis avec une solution saturée de NaCl. La phase organique est séchée sur $MgSO_4$, filtrée et concentrée sous vide. Le résidu précipite dans un mélange acétone/éther pour fournir 2,8 g du chlorure de (+) 1-[2-[3-(3,4-dichlorophényl)-1-[(3-isopropoxyphényl)acétyl]-3-pipéridinyl]éthyl]-4-phényl-1-azoniabicyclo[2.2.2]octane optiquement pur (Composé 4). F = 132°C.

$[\alpha]_D^{20}$ = + 16,3° (c = 1, dans $CH_3OH$)

EXEMPLE 5

(I) : Ar = 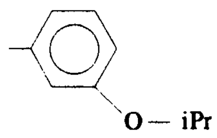 ; T = $-CH_2-$ ; R + Q = $-(CH_2)_3-$

Ar' = 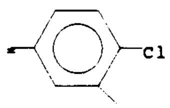 ; Am$^{\oplus}$ = 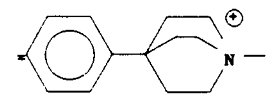 ; A$^{\ominus}$ = 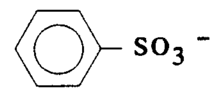

2,65 g de 4-phénylquinuclidine et 8,3 g du benzènesulfonate préparé selon la PREPARATION VII (c) sont mis en solution dans 40 ml d'acétonitrile et le mélange réactionnel est chauffé à reflux pendant 6 heures. On concentre le mélange sous vide, reprend le résidu dans le $CH_2Cl_2$ et lave successivement avec une solution aqueuse d'acide benzènesulfonique à 1 % puis à l'eau. La phase organique est séchée sur $Na_2SO_4$, filtrée et concentrée sous vide. Le résidu précipite dans l'éther isopropylique pour fournir 8 g de benzènesulfonate de (+) 1-[2-[3-(3,4-dichlorophényl)-1-[(3-isopropoxyphényl)acétyl]-3-pipéridinyl]éthyl]-4-phényl-1-azoniabicyclo[2.2.2]octane optiquement pur (Composé 5). F = 195,5°C.

$[\alpha]_D^{20}$ = -50,7° (c = 1, dans $CH_3OH$)

En procédant selon les EXEMPLES 1 à 5 on prépare les composés 6 à 12 décrits dans les TABLEAUX I et II ci-après.

TABLEAU I

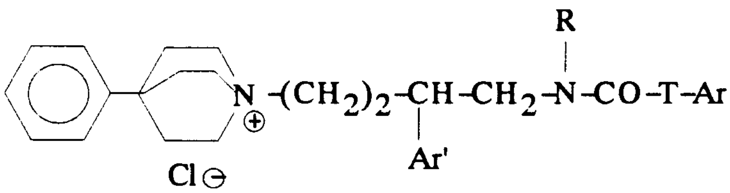

| Exemple n° | Ar' | R | T | Ar | F ; en °C et/ou $[\alpha]_D$ * |
|---|---|---|---|---|---|
| 6 | 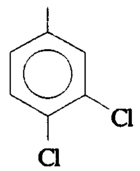 | $CH_3$ | - | 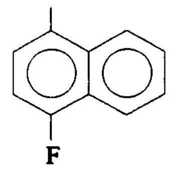 | 150<br>-57,9° |
| 7 | 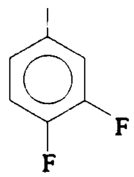 | $CH_3$ | - | 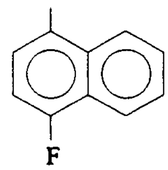 | 178 |
| 8 | 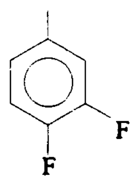 | $CH_3$ | $-CH_2-$ | 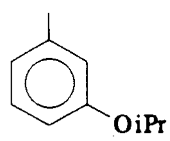 | 97 |
| 9 | 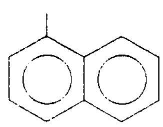 | H | - | 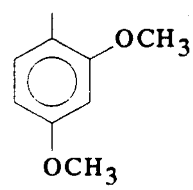 | 104-106 |

* Les pouvoirs rotatoires, $[\alpha]_D$, ont été mesurés à 20°C, c=1 dans $CH_3OH$.

TABLEAU II

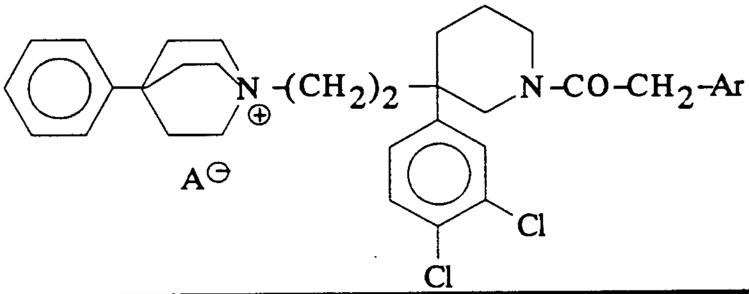

| Exemple n° | Ar | $A^{\ominus}$ | F ; °C et/ou $[\alpha]_D$ |
|---|---|---|---|
| 10 | 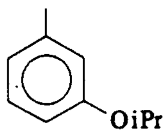 | $Cl^{\ominus}$ | 125 ; −16,0° |
| 11 | 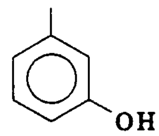 | $Br^{\ominus}$ | 196−198 |
| 12 | 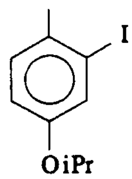 | $Cl^{\ominus}$ | 156−160<br>+11,0° |

EXEMPLE 13

(I) : Ar = 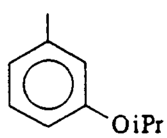 −;T = $-CH_2-$ ; Q = H .

R = $-CH_2-CH_2-O\ CO\ CH_3$ . Ar' = 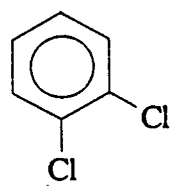

$Am^+$ = 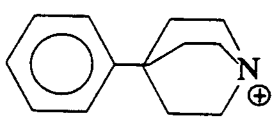 ;$A^-$ = $Cl^-$

Etape 1

8,2 g de chlorure d'éthyloxalyle sont ajoutés goutte à goutte à une solution de 19 g de 2-(2-tétrahydropyranyloxyéthyl)-3,4-dichlorobenzèneéthanamine (obtenus selon la PREPARATION (I) - Etape (b)) et de 7 g de triéthylamine. On agite le mélange réactionnel pendant une heure à température ambiante et concentre sous vide. Le résidu est repris dans l'éther éthylique, puis successivement on lave à l'eau, sèche sur $Na_2SO_4$ et concentre sous vide. Le résidu est chromatographié sur gel de silice, éluant : $CH_2Cl_2$ / $CH_3OH$ 100/1 (v/v) et on obtient 16 g de N-éthyloxalyle-2-(2-tétrahydropyranyloxyéthyl)-3,4-dichlorobenzèneéthanamine

Etape 2

16 g du produit obtenu précédemment sont mis en solution dans 40 ml de THF et ajoutés goutte à goutte à une suspension de 1,7 g de $LiAlH_4$ dans 5 ml de THF à 50°C. Le mélange réactionnel est chauffé à reflux pendant 4 heures, puis refroidi, hydrolysé, filtré et concentré sous vide. Le résidu est chromatographié sur gel de silice, éluant $CH_2Cl_2$ / $CH_3OH$ 100/5 (v/v). On obtient 14 g de N-(2-hydroxyéthyl)-2-(2-tétrahydropyranyloxyéthyl)-3,4-dichlorobenzèneéthanamine sous forme d'une huile.

Etape 3

3,55 g de BOP sont ajoutés à O°C à une solution de 2,4 g du produit préparé précédemment, 1,1 g de triéthylamine et 1,3 g d'acide 3-isopropoxyphénylacétique dans 60 ml de $CH_2Cl_2$. Le mélange réactionnel est agité pendant une heure à O°C puis successivement concentré sous vide, repris à l'AcOEt, lavé à l'eau, séché sur $Na_2SO_4$ et concentré sous vide. Le résidu est chromatographié sur gel de silice, éluant : $CH_2Cl_2$ / $CH_3OH$ 100/3 (v/v) pour fournir 2,2 g de N-(2-hydroxyéthyl)-N-3-isopropoxyphénylacétyle-2-(2-tétrahydropyranyloxyéthyl)-3,4-dichlorobenzèneéthanamine sous forme d'une huile.

Etape 4

0,41 g de chlorure d'acétyle sont ajoutés à une solution de 2,2 g du produit obtenu précédemment dans 10 ml de $CH_2Cl_2$ en présence de 0,56 g de triéthylamine dans le $CH_2Cl_2$. On agite le mélange réactionnel pendant une heure puis successivement concentre sous vide, lave à l'éther éthylique, à l'eau, sèche sur $Na_2SO_4$ et concentre sous vide pour obtenir 2 g de N-(2-acétoxyéthyl)-N-3-isopropoxyphénylacétyle-2-(2-tétrahydropyranyloxyéthyl)-3,4-dichlorobenzène-éthanamine sous forme d'une huile.

Etape 5

2 g de l'huile obtenue précédemment sont mis en solution dans 20 ml de méthanol saturé d'HCl et on agite le mélange pendant une heure à température ambiante. On concentre sous vide, reprend le résidu dans l'AcOEt, lave à l'eau, sèche sur $Na_2SO_4$ et chromatographie le résidu un gel de silice, éluant : $CH_2Cl_2$ / $CH_3OH$ 100/3 (v/v) pour obtenir 1,2 g de N-(2-acétoxyéthyl)-N-(3-isopropoxyphénylacétyl)-2-hydroxyéthyl-3,4-dichloro-benzèneéthanamine sous forme d'huile.

Etape 6

0,5 g du produit préparé précédemment sont mis en solution dans 10 ml de $CH_2Cl_2$ en présence de 0,11 g de triéthylamine. On ajoute 0,125 g de chlorure de mésyle et agite le mélange réactionnel pendant 30 minutes à température ambiante. On concentre sous vide puis le résidu est successivement repris dans l'AcOEt, lavé à l'eau, séché sur $Na_2SO_4$, concentré sous vide pour fournir 0,5 g de N-(2-acétoxyéthyl)-N-(3-isopropoxyphénylacétyl)-2-mésyloxyéthyl-3,4-dichlorobenzèneéthanamine sous forme d'huile.

Etape 7

0,50 g du produit préparé précédemment et 0,25 g de 4-phénylquinuclidine sont mis en solution dans 1 ml de diméthylformamide et on chauffe le mélange réactionnel à 80°C pendant deux heures. Le mélange réactionnel est versé dans l'eau puis successivement on extrait à l'AcOEt, lave à l'eau, lave avec une solution saturée de NaCl, concentre sous vide et chromatographie le résidu sur gel de silice, éluant : $CH_2Cl_2$ / $CH_3OH$ 100/5 (v/v). Les fractions pures sont concentrées sous vide, le résidu est repris dans $CH_2Cl_2$ et précipite par addition d'éther éthylique pour fournir 0,45 g de chlorure de 1-[3-(3,4-dichlorophényl)-4-(N-(2-acétoxyéthyl)-3-isopropoxyphénylacétylamino)butyl]-1-azoniabicyclo[2.2.2] octane (Composé 13). F = 90 - 92°C.

EXEMPLE 14

Comprimé comprenant :

| | |
|---|---|
| Composé 4 | 250 mg |
| Lactose | 80 mg |
| Polyvidone réticulée | 20 mg |
| Méthylhydroxypropylcellulose | 10 mg |
| Huile de ricin hydrogénée | 40 mg |

EXEMPLE 15

Comprimé entérique comprenant :

Comprimé :

| | |
|---|---|
| Composé 4 | 250 mg |
| Hydroxypropylcellulose | 6 mg |
| Lactose | 62 mg |
| Cellulose microcristalline | 60 mg |
| Carboxyméthylamidon | 12 mg |
| Polyéthylèneglycol 6000 | 10 mg |

Enrobage :

| | |
|---|---|
| Endraget L 100 | 1 mg |
| Phtalate de dibutyle | 1 mg |
| Alcool isopropylique (évaporé) | 28 mg |

EXEMPLE 16

Solution buvable comprenant :

| | |
|---|---|
| Composé 4 | 100 mg |
| Alcool éthylique | 100 mg |
| Propylèneglycol | 50 mg |
| Polyvidone excipient | 20 mg |
| Glycérine | 50 mg |
| Arôme artificiel | 2,5 mg |
| Eau purifié q.s.p. | 1,0 mg |

EXEMPLE 17

Suspension injectable contenant :

| | |
|---|---|
| Composé 4 | 50 mg |
| Polysorbate 80 | 1,5 mg |
| Polyoxyéthylèneglycol | 20 mg |
| Parahydroxybenzoate de méthyle et propyle | 1,5 mg |
| Sorbitol | 30 mg |
| Polyvidone excipient | 10 mg |
| Eau pour préparation injectable q.s.p | 1 mg |

EXEMPLE 18

Gélule comprenant :

| | |
|---|---|
| Composé 4 | de 2,5 à 250 mg |
| Amidon de maïs modifié | 50 mg |
| Talc | 25 mg |
| Silice colloïdale anhydre | 1 mg |
| Acide stéarique | 10 mg |
| Lactose q.s.à | 100 mg |
| Gélule | |

EXEMPLE 19

Suppositoire comprenant :

| | | |
|---|---|---|
| Composé 4 | 150 | mg |
| Glycérides semi synthétiques solides q.s.p. | | |

## Revendications

**1.** Amide basique quaternaire de formule :

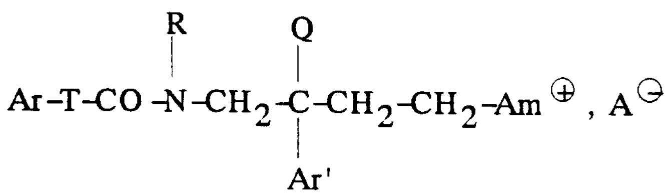

(I)

dans laquelle

- Ar représente un groupe phényle non substitué ou substitué par un ou plusieurs substituants choisis parmi : F; Cl ; Br ; I ; CN ; OH ; $NH_2$ ; $NH-CONH_2$ ; $NO_2$ ; $CONH_2$ ; $CF_3$ ; alkyle en $C_1$-$C_{10}$ ; alcényle contenant de 2 à 10 atomes de carbone; alcynyle contenant de 2 à 10 atomes de carbone ; cycloalkyle contenant de 3 à 8 atomes de carbone; bicycloalkyle contenant de 4 à 11 atomes de carbone ; hydroxyalkyle contenant de 1 à 5 atomes de carbone ; alcoxy contenant de 1 à 10 atomes de carbone ; alcoxyalkyle contenant de 2 à 10 atomes de carbone ; alcoxyalcoxyalkyle contenant jusqu'à 10 atomes de carbone ; alcoxyalcoxy contenant de 2 à 10 atomes de carbone ; alcényloxy contenant de 2 à 10 atomes de carbone ; alcényloxyalkyle avec jusqu'à 10 atomes de carbone ; alcynyloxy contenant de 2 à 10 atomes de carbone ; alcynyloxyalkyle contenant de 3 à 10 atomes de carbone ; cycloalcoxy contenant de 3 à 8 atomes de carbone ; alkylthio contenant de 1 à 10 atomes de carbone ; alkylthioalkyle contenant de 2 à 10 atomes de carbone ; acylamino contenant de 1 à 7 atomes de carbone ; acylaminoalkyle contenant de 2 à 8 atomes de carbone ; acyloxy contenant de 1 à 6 atomes de carbone ; alcoxycarbonyle contenant de 2 à 5 atomes de carbone ; cycloalcoxycarbonyle contenant de 4 à 8 atomes de carbone; alkylaminocarbonylamino contenant de 2 à 4 atomes de carbone ; dialkylaminocarbonylamino contenant de 3 à 7 atomes de carbone ; (1-pyrrolidino)carbonylamino ; cycloalkylaminocarbonylamino contenant de 4 à 8 atomes de carbone ; alkylaminocarbonylaminoalkyle contenant de 3 à 9 atomes de carbone ; dialkylaminocarbonylaminoalkyle contenant de 4 à 11 atomes de carbone ; (1-pyrrolidino)carbonylaminoéthyle ; (1-pipéridino)carbonylaminoéthyle ; cycloalkylaminocarbonylaminoalkyle contenant de 5 à 12 atomes de carbone ; alcoxycarbonylaminoalkyle contenant de 3 à 12 atomes de carbone ; cycloalcoxycarbonylaminoalkyle contenant de 5 à 12 atomes de carbone ; carbamoylalkyle contenant de 2 à 5 atomes de carbone ; alkylaminocarbonylalkyle contenant de 3 à 9 atomes de carbone ; dialkylaminocarbonylalkyle contenant de 4 à 11 atomes de carbone ; (1-pyrrolidino)carbonylméthyle; (1-pipéridino)carbonylméthyle ; (1-pipéridino)carbonyléthyle ; cycloalkylaminocarbonylalkyle contenant de 5 à 12 atomes de carbone ; alkylaminocarbonylalcoxy contenant de 3 à 10 atomes de carbone ; dialkylaminocarbonylalcoxy contenant de 4 à 10 atomes de carbone; (pipéridinyl-1)carbonylméthoxy ; cycloalkylaminocarbonylalcoxy contenant de 5 à 11 atomes de carbone ;
  ou Ar représente un groupe 1- ou 2-naphtyle; 1-, 2-, 3-, 4-, 5-, 6-, 7-indényle ; dont une ou plusieurs liaisons peuvent être hydrogénées, lesdits groupes pouvant être non substitués ou substitués par un ou plusieurs substituants choisis parmi : un halogène, les groupes alkyle, phényle, cyano, hydroxyalkyle, hydroxy, alcoxy, oxo, alkylcarbonylamino, alcoxycarbonyle et thioalkyle dans lesquels les alkyles sont en $C_1$-$C_4$; un groupe pyridyle, thiadiazolyle, indolyle, indazolyle, imidazolyle, benzimidazolyle, quinoléyle, benzotriazolyle, benzofuranyle, benzothiényle, benzothiazolyle, benzisothiazolyle, isoquinolyle, benzoxazolyle, benzoxazinyle, benzodioxinyle, isoxazolyle, benzopyranyle, thiazolyle, thiényle, furyle, pyranyle, chroményle, isobenzofurannyle, pyrrolyle, pyrazolyle, pyrazinyle, pyrimidinyle, pyridazinyle, indolizinyle, phtalazinyle, quinazolinyle, acridinyle, isothiazolyle, isochromanyle, chromanyle, dont une ou plusieurs doubles liaisons peuvent être hydrogénées, lesdits groupes pouvant être non substitués ou substitués par un ou plusieurs substituants choisis parmi : les groupes alkyle, phényle, cyano, hydroxyalkyle, hydroxy, alkylcarbonylamino, alcoxycarbonyle et thioalkyle dans lesquels les alkyles sont en $C_1$-$C_4$.

- T représente une liaison directe, un groupe hydroxyméthylène, un groupe alcoxyméthylène dont le groupe alcoxy est en $C_1$-$C_4$ ou un groupe alkylène en $C_1$-$C_5$ ;
- Ar' représente un phényle non substitué ou substitué une ou plusieurs fois par un des substituants choisis parmi : un atome d'halogène, un trifluorométhyle, un alcoxy en $C_1$-$C_4$, un alkyle en $C_1$-$C_4$, lesdits substituants étant identiques ou différents ; un thiényle ; un benzothiényle ; un naphtyle ou un indolyle ;
- R représente l'hydrogène ; un alkyle en $C_1$-$C_4$ ; un ω-($C_1$-$C_4$)alcoxy($C_2$-$C_4$)alkyle ; ou un ω-($C_2$-$C_4$)alcanoyloxy($C_2$-$C_4$)alkyle ;
- Q représente l'hydrogène ;
- ou bien Q et R, ensemble, forment un groupe 1,2-éthylène, 1,3-propylène ou 1,4-butylène ;
- $Am^{\oplus}$ représente le radical

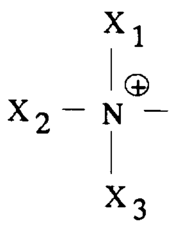

dans lequel $X_1$, $X_2$, $X_3$, ensemble et avec l'atome d'azote auquel ils sont liés, forment un système azabicyclique choisi parmi 1-azoniabicyclo[2.2.1]heptane et 1-azoniabicyclo[2.2.2]octane, éventuellement substitué par un groupe phényle ou benzyle ;
- $A^{\ominus}$ est un anion pharmaceutiquement acceptable.

**2.** Amide basique quaternaire de formule (I) selon la revendication 1 dans laquelle $Am^{\oplus}$ représente un groupe 1-azoniabicyclo[2.2.2]octane ou un groupe 4-phényl-1-azoniabicyclo[2.2.2]octane.

**3.** Amide basique quaternaire de formule (I) selon l'une des revendications 1 ou 2 dans laquelle T, R, Ar' et Q sont tels que définis pour (I) et $Am^{\oplus}$ représente un radical 1-azoniabicyclo[2.2.2]octane ou 4-phényl-1-azoniabicyclo[2.2.2]octane, Ar représente un phényle substitué par un alcoxy en $C_1$-$C_4$ et $A^{\ominus}$ est un anion pharmaceutiquement acceptable.

**4.** Amide basique quaternaire selon la revendication 1 de formule :

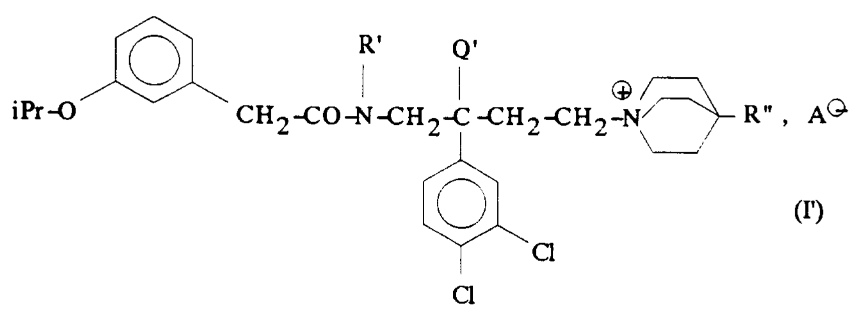

(I')

dans laquelle R' et Q' représentent, respectivement, un groupe méthyle et l'hydrogène, un groupe 2-acétoxyéthyle et l'hydrogène ou, ensemble, forment un groupe 1,3-propylène, R" est l'hydrogène ou un groupe phényle et $A^{\ominus}$ est un anion pharmaceutiquement acceptable.

**5.** Amide basique quaternaire selon la revendication 1 de formule :

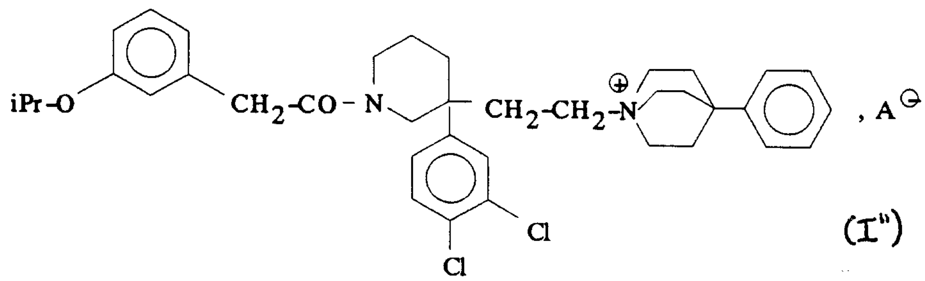

(I")

dans laquelle $A^{\ominus}$ est un anion pharmaceutiquement acceptable.

6. Amide basique quaternaire selon l'une quelconque des revendications 1 à 5, caractérisé en ce que $A^{\ominus}$ est un anion choisi parmi chlorure, bromure, iodure, hydrogénosulfate, méthanesulfonate, paratoluènesulfonate, benzènesulfonate et acétate.

7. Amide basique quaternaire selon la revendication 1 qui est le chlorure de (+) 1-[2-[3-(3,4-dichlorophényl)-1-[(3-isopropoxyphényl)acétyl]-3-pipéridinyl]éthyl]-4-phényl-1-azoniabicyclo[2.2.2]octane.

8. Procédé pour la préparation des composés de formule (I) selon la revendication 1, caractérisé en ce qu'on traite un dérivé de formule :

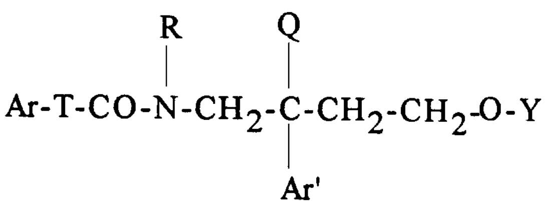

(II)

dans lequel OY représente un groupe partant, avec une amine tertiaire cyclique de formule :

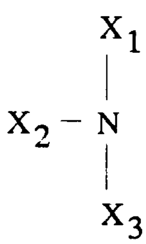

(III)

dans un solvant aprotique polaire à une température comprise entre la température ambiante et 120°C, puis éventuellement on échange l'anion du sel quaternaire ainsi obtenu avec un autre anion pharmaceutiquement acceptable.

9. Procédé selon la revendication 8, caractérisé en ce que le groupe partant OY est un groupe méthanesulfonate ou benzènesulfonate.

10. Procédé selon l'une des revendications 8 ou 9, caractérisé en ce que l'amine tertiaire cyclique (III) est choisie parmi :

(c') 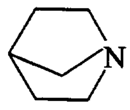 (d') 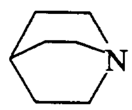 (l') 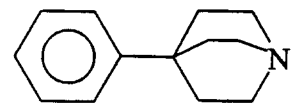

**11.** Composition pharmaceutique contenant en tant que principe actif, un composé de formule (I) selon la revendication 1.

**12.** Composition pharmaceutique selon la revendication 11, sous forme d'unité de dosage, dans laquelle le principe actif est mélangé à au moins un excipient pharmaceutique.

**13.** Composition selon la revendication 12 contenant de 0,5 à 1000 mg de principe actif.

**14.** Composition selon la revendication 13 contenant de 2,5 à 250 mg de principe actif.

**Claims**

**1.** A quaternary basic amide of the formula:

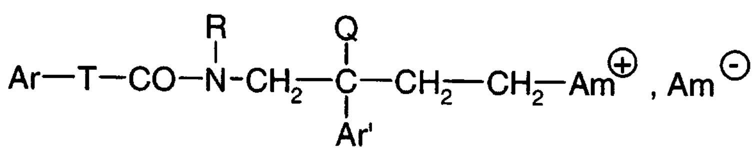
(I)

in which

- Ar is a phenyl group that is unsubstituted or substituted by one or more substituents selected from: F; Cl; Br; I; CN; OH; $NH_2$; NH-$CONH_2$; $NO_2$; $CONH_2$; $CF_3$; $C_1$-$C_{10}$-alkyl; alkenyl containing from 2 to 10 carbon atoms; alkynyl containing from 2 to 10 carbon atoms; cycloalkyl containing from 3 to 8 carbon atoms; bicycloalkyl containing from 4 to 11 carbon atoms; hydroxyalkyl containing from 1 to 5 carbon atoms; alkoxy containing from 1 to 10 carbon atoms; alkoxyalkyl containing from 2 to 10 carbon atoms; alkoxyalkoxyalkyl containing up to 10 carbon atoms; alkoxyalkoxy containing from 2 to 10 carbon atoms; alkenyloxy containing from 2 to 10 carbon atoms; alkenyloxyalkyl having up to 10 carbon atoms; alkynyloxy containing from 2 to 10 carbon atoms; alkynyloxyalkyl containing from 3 to 10 carbon atoms; cycloalkoxy containing from 3 to 8 carbon atoms; alkylthio containing from 1 to 10 carbon atoms; alkylthioalkyl containing from 2 to 10 carbon atoms; acylamino containing from 1 to 7 carbon atoms; acylaminoalkyl containing from 2 to 8 carbon atoms; acyloxy containing from 1 to 6 carbon atoms; alkoxycarbonyl containing from 2 to 5 carbon atoms; cycloalkoxycarbonyl containing from 4 to 8 carbon atoms; alkylaminocarbonylamino containing from 2 to 4 carbon atoms; dialkylaminocarbonylamino containing from 3 to 7 carbon atoms; (pyrrolidin-1-yl)carbonylamino; cycloalkylaminocarbonylamino containing from 4 to 8 carbon atoms; alkylaminocarbonylaminoalkyl containing from 3 to 9 carbon atoms; dialkylaminocarbonylaminoalkyl containing from 4 to 11 carbon atoms; (pyrrolidin-1-yl)carbonylaminoethyl; (piperidin-1-yl)carbonylaminoethyl; cycloalkylaminocarbonylaminoalkyl containing from 5 to 12 carbon atoms; alkoxycarbonylaminoalkyl containing from 3 to 12 carbon atoms; cycloalkoxycarbonylaminoalkyl containing from 5 to 12 carbon atoms; carbamoylalkyl containing from 2 to 5 carbon atoms; alkylaminocarbonylalkyl containing from 3 to 9 carbon atoms; dialkylaminocarbonylalkyl containing from 4 to 11 carbon atoms; (pyrrolidin-1-yl)carbonylmethyl; (piperidin-1-yl)carbonylmethyl; (piperidin-1-yl)carbonylethyl; cycloalkylaminocarbonylalkyl containing from 5 to 12 carbon atoms; alkylaminocarbonylalkoxy containing from 3 to 10 carbon atoms; dialkylaminocarbonylalkoxy containing from 4 to 10 carbon atoms; (piperidin-1-yl)carbonylmethoxy; cycloalkylaminocarbonylalkoxy containing from 5 to 11 carbon atoms;
  or Ar is a 1- or 2-naphthyl group; a 1-, 2-, 3-, 4-, 5-, 6-, 7-indenyl group; in which one or more bonds can be

hydrogenated, it being possible for said groups to be unsubstituted or substituted by one or more substituents selected from: a halogen, the alkyl, phenyl, cyano, hydroxyalkyl, hydroxy, alkoxy, oxo, alkylcarbonylamino, alkoxycarbonyl and thioalkyl groups in which the alkyls are $C_1$-$C_4$; a pyridyl, thiadiazolyl, indolyl, indazolyl, imidazolyl, benzimidazolyl, quinolyl, benzotriazolyl, benzofuranyl, benzothienyl, benzothiazolyl, benzisothiazolyl, isoquinolyl, benzoxazolyl, benzoxazinyl, benzodioxinyl, isoxazolyl, benzopyranyl, thiazolyl, thienyl, furyl, pyranyl, chromenyl, isobenzofuranyl, pyrrolyl, pyrazolyl, pyrazinyl, pyrimidinyl, pyridazinyl, indolizinyl, phthalazinyl, quinazolinyl, acridinyl, isothiazolyl, isochromanyl or chromanyl group, in which one or more double bonds can be hydrogenated, it being possible for said groups to be unsubstituted or substituted by one or more substituents selected from: alkyl, phenyl, cyano, hydroxyalkyl, hydroxy, alkylcarbonylamino, alkoxycarbonyl and thioalkyl groups in which the alkyls are $C_1$ -$C_4$.

- T is a direct bond, a hydroxymethylene group, an alkoxymethylene group in which the alkoxy group is $C_1$-$C_4$, or a $C_1$-$C_5$-alkylene group;
- Ar' is a phenyl which is unsubstituted or mono- or polysubstituted by one of the substituents selected from: a halogen atom, a trifluoromethyl, a $C_1$ -$C_4$-alkoxy, a $C_1$ -$C_4$-alkyl, said substituents being identical or different; a thienyl; a benzothienyl; a naphthyl or an indolyl;
- R is hydrogen; a $C_1$ -$C_4$-alkyl; a ($C_1$ -$C_4$)-ω-alkoxy($C_2$ -$C_4$)alkyl; or a ($C_2$ -$C_4$)-ω-alkanoyloxy($C_2$ -$C_4$)alkyl;
- Q is hydrogen;
- or else Q and R together form a 1,2-ethylene, 1,3- propylene or 1,4-butylene group;
- $Am^{\oplus}$ is the radical

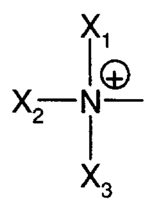

in which $X_1$, $X_2$, $X_3$, together with the nitrogen atom to which they are bonded, form an azabicyclic system selected from 1-azoniabicyclo[2.2.1]heptane and 1-azoniabicyclo[2.2.2]octane, optionally substituted by a phenyl or benzyl group;

- $A^{\ominus}$ is a pharmaceutically acceptable anion.

**2.** A quaternary basic amide of formula (I) according to claim 1 in which $Am^{\oplus}$ is a 1-azoniabicyclo[2.2.2]octane group or a 4-phenyl-1-azoniabicyclo[2.2.2]octane group.

**3.** A quaternary basic amide of formula (I) according to one of claims 1 or 2 in which T, R, Ar' and Q are as defined for (I) and
$Am^{\oplus}$ is a 1-azoniabicyclo[2.2.2]octane or 4-phenyl-1-azoniabicyclo[2.2.2]octane radical, Ar is a phenyl substituted by a $C_1$ -$C_4$-alkoxy and $A^{\ominus}$ is a pharmaceutically acceptable anion.

**4.** A quaternary basic amide according to claim 1 of the formula:

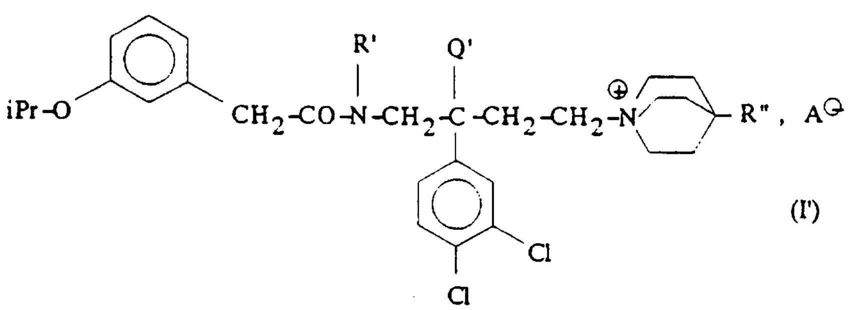

(I')

in which R' and Q' are respectively a methyl group and hydrogen, a 2-acetoxyethyl group and hydrogen, or R' and

Q' form together a 1,3-propylene group, R'' is hydrogen or a phenyl group and $A^{\ominus}$ is a pharmaceutically acceptable anion.

**5.** A quaternary basic amide according to claim 1 of the formula:

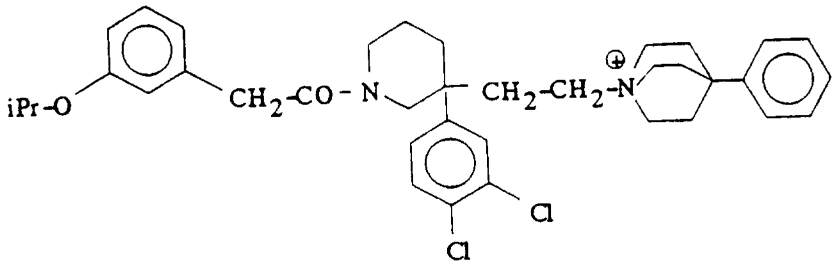

, $A^{\ominus}$

I''

in which $A^{\ominus}$ is a pharmaceutically acceptable anion.

**6.** A quaternary basic amide according to any one of claims 1 to 5, characterised in that $A^{\ominus}$ is an anion selected from chloride, bromide, iodide, hydrogensulfate, methanesulfonate, paratoluenesulfonate, benzenesulfonate and acetate.

**7.** A quaternary basic amide according to claim 1 which is the (+)-1-[2-[3-(3,4-Dichlorophenyl)-1-[(3-isopropoxyphenyl)acetyl]piperidin-3-yl]ethyl]-4-phenyl-1-azoniabicyclo[2.2.2]octane chloride.

**8.** A method of preparing the compounds of formula (I) according to claim 1, characterised in that a derivative of the formula:

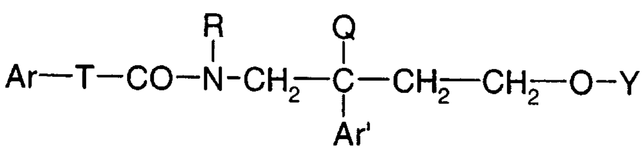

(II)

is treated, in which OY represents a leaving group, with a cyclic tertiary amine of the formula:

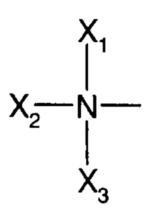

(III)

in a polar aprotic solvent, at a temperature between room temperature and 120°C, and then, if appropriate, the anion of the resulting quaternary salt is exchanged with another pharmaceutically acceptable anion.

**9.** A method according to claim 8, characterised in that the leaving group OY is a methanesulfonate or benzenesulfonate group.

**10.** A method according to one of claims 8 or 9, characterised in that the cyclic tertiary amine (III) is selected from:

(c') 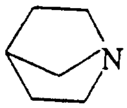 (d') 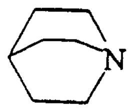 (l') 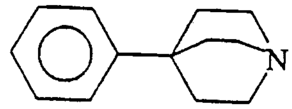

**11.** A pharmaceutical composition in which a compound of formula (I) according to claim 1 is present as the active principle.

**12.** A pharmaceutical composition according to claim 11, in the form of a dosage unit in which the active principle is mixed with at least one pharmaceutical excipient.

**13.** A composition according to claim 12 containing from 0.5 to 1,000 mg of active principle.

**14.** A composition according to claim 13 containing from 2.5 to 250 mg of active principle.

**Patentansprüche**

**1.** Quartäre basische Amide der Formel:

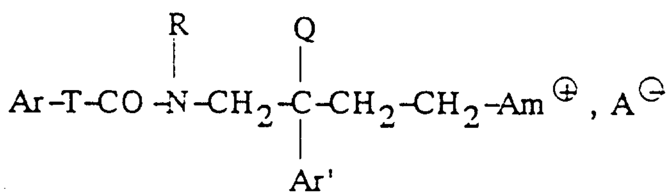 (I),

in der bedeuten:

- Ar eine Phenylgruppe, die nicht substituiert ist oder mit einem oder mehreren Substituenten substituiert ist, die ausgewählt sind unter: F, Cl, Br, I, CN, OH, $NH_2$, NH-$CONH_2$, $NO_2$, $CONH_2$, $CF_3$, $C_{1-10}$-Alkyl, Alkenyl mit 2 bis 10 Kohlenstoffatomen, Alkinyl mit 2 bis 10 Kohlenstoffatomen, Cycloalkyl mit 3 bis 8 Kohlenstoffatomen, Bicycloalkyl mit 4 bis 11 Kohlenstoffatomen, Hydroxyalkyl mit 1 bis 5 Kohlenstoffatomen, Alkoxy mit 1 bis 10 Kohlenstoffatomen, Alkoxyalkyl mit 2 bis 10 Kohlenstoffatomen, Alkoxyalkoxyalkyl mit bis zu 10 Kohlenstoffatomen, Alkoxyalkoxy mit 2 bis 10 Kohlenstoffatomen, Alkenyloxy mit 2 bis 10 Kohlenstoffatomen, Alkenyloxyalkyl mit bis zu 10 Kohlenstoffatomen, Alkinyloxy mit 2 bis 10 Kohlenstoffatomen, Alkinyloxyalkyl mit 3 bis 10 Kohlenstoffatomen, Cycloalkoxy mit 3 bis 8 Kohlenstoffatomen, Alkylthio mit 1 bis 10 Kohlenstoffatomen, Alkylthioalkyl mit 2 bis 10 Kohlenstoffatomen, Acylamino mit 1 bis 7 Kohlenstoffatomen, Acylaminoalkyl mit 2 bis 8 Kohlenstoffatomen, Acyloxy mit 1 bis 6 Kohlenstoffatomen, Alkoxycarbonyl mit 2 bis 5 Kohlenstoffatomen, Cycloalkoxycarbonyl mit 4 bis 8 Kohlenstoffatomen, Alkylaminocarbonylamino mit 2 bis 4 Kohlenstoffatomen, Dialkylaminocarbonylamino mit 3 bis 7 Kohlenstoffatomen, (1-Pyrrolidino)-carbonylamino, Cycloalkylaminocarbonylamino mit 4 bis 8 Kohlenstoffatomen, Alkylaminocarbonylaminoalkyl mit 3 bis 9 Kohlenstoffatomen, Dialkylaminocarbonylaminoalkyl mit 4 bis 11 Kohlenstoffatomen, (1-Pyrrolidino)-carbonylaminoethyl, (1-Piperidino)-carbonylaminoethyl, Cycloalkylaminocarbonylaminoalkyl mit 5 bis 12 Kohlenstoffatomen, Alkoxycarbonylaminoalkyl mit 3 bis 12 Kohlenstoffatomen, Cycloalkoxycarbonylaminoalkyl bis 5 bis 12 Kohlenstoffatomen, Carbamoylalkyl mit 2 bis 5 Kohlenstoffatomen, Alkylaminocarbonylalkyl mit 3 bis 9 Kohlenstoffatomen, Dialkylaminocarbonylalkyl mit 4 bis 11 Kohlenstoffatomen, (1-Pyrrolidino)-carbonylmethyl, (1-Piperidino)-carbonylmethyl, (1-Piperidino)-carbonylethyl, Cycloalkylaminocarbonylalkyl mit 5 bis 12 Kohlenstoffatomen, Alkylaminocarbonylalkoxy mit 3 bis 10 Kohlenstoffatomen, Dialkylaminocarbonylalkoxy mit 4 bis 10 Kohlenstoffatomen, (Piperidinyl-1-)-carbonylmethoxy, Cycloalkylaminocarbonylalkoxy mit 5 bis 11 Kohlenstoffatomen, oder in der Ar bedeutet: 1- oder 2-Naphtyl, 1-, 2-, 3-, 4-, 5-, 6-, 7-Indenyl, bei denen eine oder mehrere Bindungen hydriert sein können, wobei diese Gruppen unsubstituiert oder mit einem oder mehreren Substituenten substituiert sein können, die ausgewählt sind unter: Halogen, Alkyl, Phenyl, Cyano, Hydroxyalkyl, Hydroxy, Alkoxy, Oxo, Alkylcarbonylamino, Alkoxycarbonyl und Thioalkyl, worin die Alkylgruppen 1 bis 4 Kohlenstoffatome aufweisen, Pyridyl, Thiadiazolyl, Indolyl, Indazolyl, Imidazolyl, Benzimidazolyl, Chinoleyl, Benzotriazolyl, Benzofuranyl, Benzothienyl, Benzothiazolyl, Benzisothiazolyl, Isochinolyl, Benzoxazolyl, Benzoxazinyl, Benzodioxinyl, Isoxazolyl, Benzopyranyl, Thiazolyl, Thienyl, Furyl, Pyranyl, Chromenyl, Isobenzofuranyl, Pyrrolyl, Pyrazolyl, Pyrazinyl, Pyrimidinyl, Pyridazinyl, Indolizinyl, Phthalazinyl, Chinazolinyl, Acridinyl, Isothiazolyl, Isochromanyl, Chromanyl, bei denen eine oder mehrere Doppelbindungen hydriert sein können, wobei diese Gruppen nicht substituiert oder mit einer oder mehreren Gruppen substituiert sein können, die ausgewählt sind unter: Alkyl, Phenyl, Cyano, Hydroxyalkyl, Hydroxy, Alkylcarbonylamino, Alkoxycarbonyl und Thioalkyl, worin die Alkylgruppen 1 bis 4 Kohlenstoffatome aufweisen,

- T eine direkte Bindung, Hydroxymethylen, Alkoxymethylen, wobei die Alkyoxygruppe 1 bis 4 Kohlenstoffatome aufweist, oder $C_{1-5}$-Alkylen,
- Ar' nicht substituiertes Phenyl oder Phenyl, das mit einer oder mehreren Substituenten substituiert ist, die unter Halogen, Trifluormethyl, $C_{1-4}$-Alkoxy, $C_{1-4}$-Alkyl, wobei die Substituenten gleich oder verschieden sind, Thienyl, Benzothienyl, Naphtyl und Indolyl ausgewählt sind,
- R Wasserstoff, $C_{1-4}$-Alkyl, ω-($C_{1-4}$)-Alkoxy-($C_{2-4}$)-alkyl oder ω-($C_{2-4}$)-Alkanoyloxy-($C_{2-4}$)-alkyl
- Q Wasserstoff,
- oder Q und R zusammen 1,2-Ethylen, 1,3-Propylen oder 1,4-Butylen,
- $Am^+$ die Gruppe

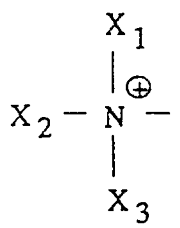

in der $X_1$, $X_2$, $X_3$ zusammen mit dem Stickstoffatom, mit dem sie verbunden sind, ein azabicyclisches System bilden, das unter 1-Azoniabicyclo[2.2.1]heptan und 1-Azoniabicyclo[2.2.2]octan ausgewählt ist, das gegebenenfalls mit einer Phenyl- oder Benzylgruppe substituiert ist,

- $A^-$ ein pharmazeutisch akzeptables Anion.

**2.** Quartäres basisches Amid der Formel (I) nach Anspruch 1, in der $Am^+$ 1-Azoniabicyclo[2.2.2]octan oder 4-Phenyl-1-azoniabicyclo[2,2,2]octan darstellt.

**3.** Quartäres basisches Amid der Formel (I) nach einem der Ansprüche 1 und 2, in der T, R, Ar' und Q wie für (I) definiert sind und $Am^+$ 1-Azoniabicyclo[2.2.2]octan oder 4-Phenyl-1-azoniabicyclo[2.2.2]octan, Ar ein mit einer $C_{1-4}$-Alkoxygruppe substituiertes Phenyl und $A^-$ ein pharmazeutisch akzeptables Anion bedeutet.

**4.** Quartäres basisches Amid nach Anspruch 1 der Formel

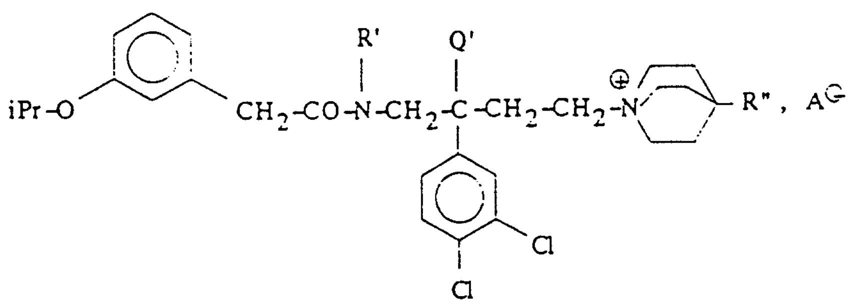

(I'),

in der R' und Q' Methyl und Wasserstoff bzw. 2-Acetoxyethyl und Wasserstoff darstellen oder zusammen eine 1,3-Propylen-Gruppe bilden, R'' Wasserstoff oder Phenyl und $A^{\ominus}$ ein pharmazeutisch akzeptables Anion ist.

**5.** Quartäres basisches Amid nach Anspruch 1 der Formel

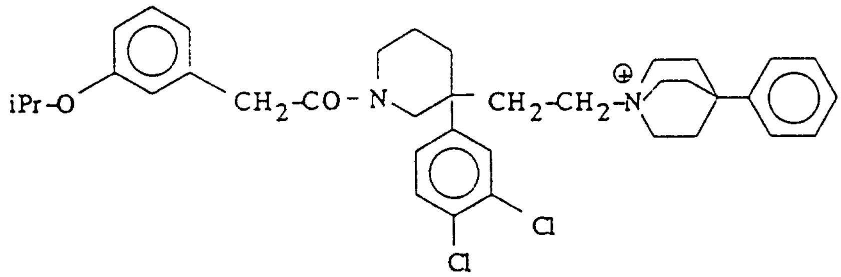 , $A^{\ominus}$ (I"),

in der $A^-$ ein pharmazeutisch akzeptables Anion ist.

**6.** Quartäres basisches Amid nach einem der Ansprüche 1 bis 5, dadurch gekennzeichnet, daß $A^-$ ein Anion ist, das unter Chlorid, Bromid, Iodid, Hydrogensulfat, Methansulfonat, p-Toluolsulfonat, Benzolsulfonat und Acetat ausgewählt ist.

**7.** Quartäres basisches Amid nach Anspruch 1, bei dem es sich um das Chlorid von (+) 1-[2-[3,4-Dichlorphenyl)-1-[(3-isopropoxyphenyl)-acetyl]-3-piperidinyl]-ethyl]-4-phenyl-1-azoniabicyclo[2.2.2]octan handelt.

**8.** Verfahren zur Herstellung der Verbindungen der Formel (I) nach Anspruch 1, dadurch gekennzeichnet, daß ein Derivat der Formel

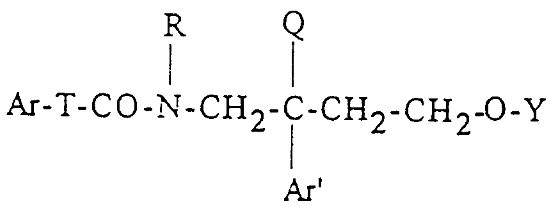 (II),

in der OY eine Abgangsgruppe darstellt, mit einem cyclischen tertiären Amin der Formel

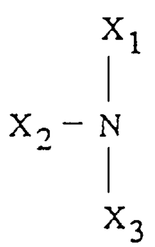 (III)

in einem polaren aprotischen Lösungsmittel bei einer Temperatur von Umgebungstemperatur bis 120 °C behandelt wird, wonach gegebenenfalls das Anion des so erhaltenen quartären Salzes durch ein anderes pharmazeutisch akzeptables Anion ausgetauscht wird.

**9.** Verfahren nach Anspruch 8, dadurch gekennzeichnet, daß die Abgangsgruppe OY eine Methansulfonat- oder Benzolsulfonatgruppe ist.

**10.** Verfahren nach einem der Ansprüche 8 oder 9, dadurch gekennzeichnet, daß das cyclische tertiäre Amin (III) ausgewählt ist unter:

(c') 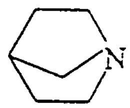 (d') 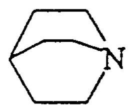 (l') 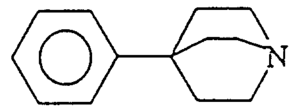

**11.** Pharmazeutische Zusammensetzung, die als Wirkstoff eine Verbindung der Formel (I) nach Anspruch 1 enthält.

**12.** Pharmazeutische Zusammensetzung nach Anspruch 11 in Form einer Dosiseinheit, in der der Wirkstoff mit mindestens einem pharmazeutischen Exzipienten vermischt ist.

**13.** Zusammensetzung nach Anspruch 12, die 0,5 bis 1000 mg Wirkstoff enthält.

**14.** Zusammensetzung nach Anspruch 13, die 2,5 bis 250 mg Wirkstoff enthält.